# EUROPEAN PATENT APPLICATION

(11) **EP 0 731 107 A1**
(43) Date of publication of application: **11.09.1996**
(21) Application number: 96101952.8
(22) Date of filing: 10.02.1996
(51) Int. Cl.: C07K 5/06

(54) **Production of aldehyde derivatives**

(30) Priority: 13.02.1995 JP 24250/95
(71) Applicant: TAKEDA CHEMICAL INDUSTRIES, LTD., Chuo-ku, Osaka 541 (JP)
(72) Inventor: Sohda, Takashi, Takatsuki, Osaka 569 (JP); Yasuma, Tsuneo, Ibaraki, Osaka 567 (JP)
(74) Representative: von Kreisler, Alek, Dipl.-Chem.

(57) **Abstract**

This invention relates to a method for producing a compound of the formula (II) 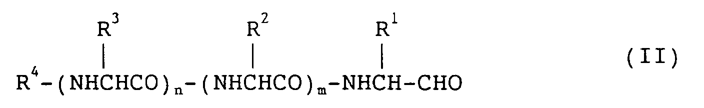 wherein R¹, R² and R³ independently represent hydrogen or a hydrocarbon group that may be substituted; R⁴ represents acyl; m and n are independently 0 or 1 or a salt thereof, which comprises subjecting a compound of the formula (I) 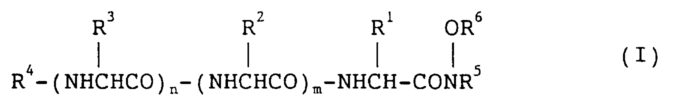 wherein R¹, R², R³, R⁴, m and n are as defined above; R⁵ and R⁶ independently represent a hydrocarbon group that may be substituted; or a salt thereof to a reduction reaction, by which the objective compound can be obtained under mild conditions and in good yield.

## Description

### Field of the Invention

The present invention relates to the production of aldehyde derivatives and salts which are of value as bone resorption inhibitors and to their synthetic intermediates.

### Background of the Invention

Peptide aldehyde derivatives are generally synthesized by subjecting the corresponding alcohols to DMSO oxidation. Thus, EP-A1-0623627 and JP Kokai H-5-163221 (EP 520336), for instance, describe that aldehyde derivatives can be synthesized by DMSO oxidation of the corresponding alcohols. According to this synthetic technology, however, the odor of dimethyl sulfide formed in DMSO oxidation is sometimes transferred to aldehyde derivatives and it is desirable that this odor be thoroughly eliminated.

Therefore, establishment of a technology for producing an optically active peptide aldehyde derivative under mild conditions and in good yield without resort to DMSO oxidation is desirable.

### Disclosure of Invention

In order to solve the above problem, the inventors of this invention did much research and discovered that an optically active aldehyde derivative which is odorless can be provided by reducing the corresponding compound of the formula (I) or a salt thereof. 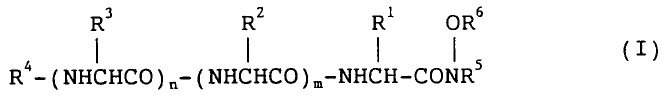 wherein R¹, R² and R³ are the same or different and each represents hydrogen or a hydrocarbon residue that may be substituted; R⁴ represents acyl; R⁵ and R⁶ are the same or different and each represents a hydrocarbon residue that may be substituted; m and n are the same or different and each is equal to 0 or 1. This invention has been developed on the basis of the above finding.

This invention, therefore, relates to
(1) a method for producing a compound of the formula (II) 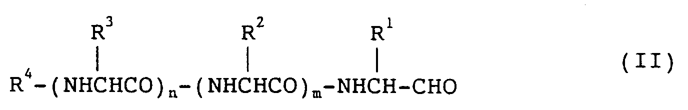 wherein R¹, R² and R³ independently represent hydrogen or a hydrocarbon group that may be substituted; R⁴ represents acyl; m and n are independently 0 or 1 or a salt thereof, which comprises subjecting a compound of the formula (I) 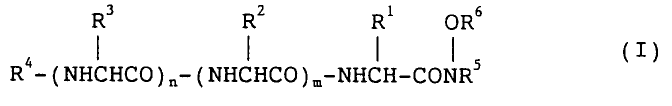 wherein R¹, R², R³, R⁴, m and n are as defined above; R⁵ and R⁶ independently represent a hydrocarbon group that may be substituted; or a salt thereof to a reduction reaction,
(2) a method mentioned in the above (1), in which the reduction reaction is carried out using an aluminum hydride type reducing agent,
(3) a method mentioned in the above (1), in which R₁, R₂ and R³ independently represent hydrogen or a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl wherein the hydrocarbon group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which may be substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen or a 5-membered ring containing 1 atom of sulfur in which the heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy; R⁴ represents a group of the formula -CONHR⁷, -CSNHR⁸, -COR⁹, -SOR¹⁰ or -SO₂R¹¹ wherein R⁷, R⁸, R⁹, R¹⁰ and R¹¹ independently represent a hydrogen atom or (A) a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl or (B) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur and oxgen which may be condensed with a 6-membered ring containing 2 or fewer atoms of nitrogen, a benzene ring or a 5-membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may be condensed with a benzene ring, a 6-membered ring containing 2 or fewer atoms of nitrogen, or a 5-membered ring containing 1 atom of sulfur which hydrocarbon or heterocyclic group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which maybe substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen, or a 5-membered ring containing 1 atom of sulfur which heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy; R⁵ and R⁶ independently represent hydrogen or a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl wherein the hydrocarbon group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which may be substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen, or a 5-membered ring containing 1 atom of sulfur which heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy,
(4) a method mentioned in the above (1), in which R¹, R² and R³ are independently an optionally substituted alkyl group,
(5) a method mentioned in the above (1), in which R¹ is a straight-chain or branched C₁₋₆ alkyl group which is substituted with an aryl group or a heterocyclic group,
(6) a method mentioned in the above (1), in which R² and R³ are independently a straight-chain or branched C₁₋₆ alkyl group,
(7) a method mentioned in the above (1), in which the acyl group is that derived from a carboxylic acid, sulfonic acid, sulfinic acid, carbamic acid or thiocarbamic acid,
(8) a method mentioned in the above (1), in which the acyl group is represented by the formula -SO₂R¹¹ or - COR⁹, wherein R¹¹ and R⁹ are independently a hydrogen atom or an optionally substituted hydrocarbon or heterocyclic group,
(9) a method mentioned in the above (1), in which R⁴ represents a group of the formula -SO₂R^{11'} wherein R^{11'} is a C₆₋₁₀ aryl group,
(10) a method mentioned in the above (1), in which R⁵ and R⁶ independently represent an alkyl,
(11) a method mentioned in the above (10) wherein the alkyl is methyl,
(12) a method mentioned in the above (1), in which m is 1 and n is 0.
(13) a compound of the formula (I) 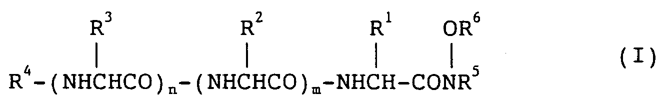 wherein R¹, R² and R³ independently represent hydrogen or a hydrocarbon group that may be substituted; R⁴ represents acyl; R⁵ and R⁶ independently represent a hydrocarbon group that may be substituted; m and n are independently 0 or 1 or a salt thereof,
(14) a compound mentioned in the above (13), which is one of the formula ( 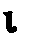 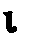 ) 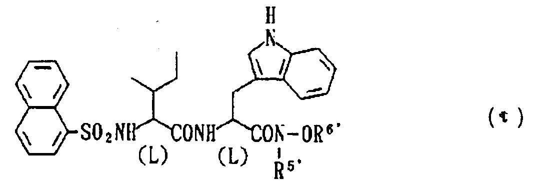 wherein R^{5'} and R^{6'} independently a C₁₋₆ alkyl group or a reactive derivative of the amino function thereof or a salt thereof, and
(15) a method for producing a compound of the formula 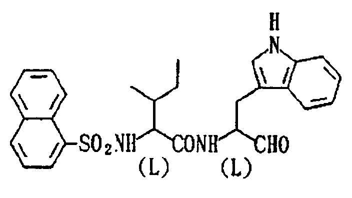 which comprises (i) reacting a compound of the formula (α) 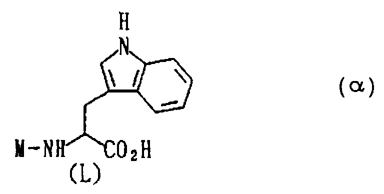 wherein M represents an amino-protecting group or a reactive derivative of the carboxyl function thereof or a salt thereof with a compound of the formula (β) 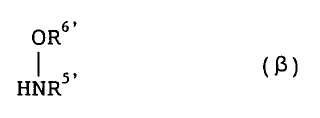 wherein R^{5'} and R^{6'} independently represent a C₁₋₆ alkyl group or a reactive derivative of the amino function thereof or a salt thereof to give a compound of the formula (γ) 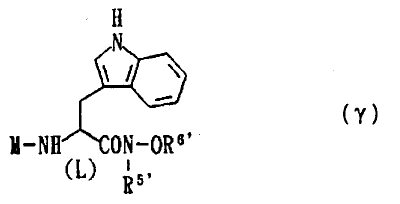 wherein R^{5'}, R^{6'} and M are as defined above and, then, subjecting the compound to the deprotection reaction of the amino protecting group to produce a compound of the formula (δ) 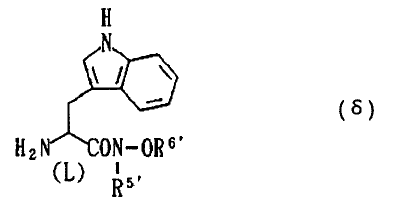 wherein R^{5'} and R^{6'} are as defined above,
   (ii) reacting the compound of the formula (δ) or a reactive derivative of the amino function thereof or a salt thereof with a compound of the formula (ε) 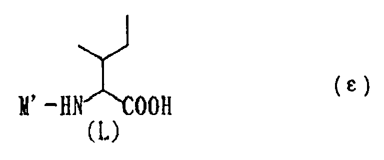 wherein M' represents an amino-protecting group or a reactive derivative of the carboxyl function thereof or a salt thereof to give a compound of the formula (ζ) 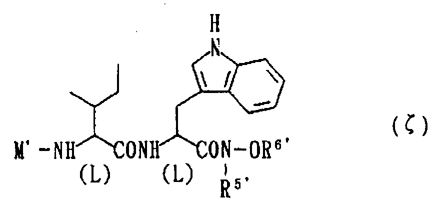 wherein R^{5'}, R^{6'} and M' are as defined above and, then, subjecting the compound to the deprotection reaction of the amino-protecting group to produce a compound of the formula (η) 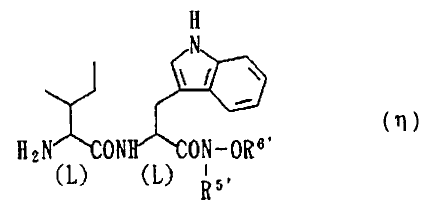 wherein R^{5'} and R⁶' are as defined above,
   (iii) reacting the compound of the formula (η) or a reactive derivative of the amino function thereof or a salt thereof with a compound of the formula (θ) 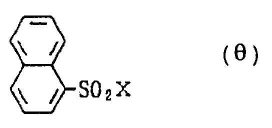 wherein X is a halogen atom, or a salt thereof to give a compound of the formula ( 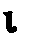 ) 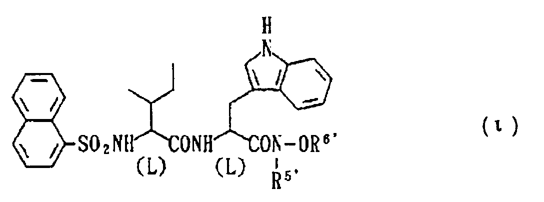 wherein R^{5'} and R^{6'} are as defined above, and
   (iv) subjecting the compound of the formula ( 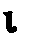 ) or a salt thereof to a reduction reaction.

Explanation of the various definitions used in the above formulas and relevant to the scope of the invention and some preferred examples of the species thereof are given below.

The constituent amino acids mentioned in this specification are L-compounds unless otherwise indicated and where represented by abbreviations these amino acids are invariably designated in conformity with the rules of nomenclature of International Union of Pure and Applied Chemistry (IUPAC)-International Union of Biochemistry (IUB).

Referring to the above formulas (I) and (II), the hydrocarbon group of said "hydrocarbon residue that may be substituted" for any of R¹, R² and R³ includes saturated and unsaturated aliphatic acyclic hydrocarbon groups, saturated and unsaturated alicyclic hydrocarbon groups, and aryl groups.

The saturated aliphatic hydrocarbon group includes but is not limited to straight-chain or branched C₁₋₁₀ saturated aliphatic hydrocarbon groups (e.g. C₁₋₁₀ alkyl groups such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.) and is preferably a straight-chain or branched C₁₋₆ saturated aliphatic hydrocarbon group.

The unsaturated aliphatic hydrocarbon group includes straight-chain and branched C₂₋₁₀ unsaturated aliphatic hydrocarbon groups (e.g. C₂₋₁₀ alkenyl such as ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, etc. and C₂₋₁₀ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 2,4-hexadiynyl, 5-hexynyl, 1-heptynyl, 1-octynyl, etc.) and, preferably, straight-chain and branched C₂₋₆ unsaturated aliphatic hydrocarbon groups.

The saturated alicyclic hydrocarbon group includes saturated C₃₋₁₂ alicyclic hydrocarbon groups (e.g. C₃₋₁₂ cycloalkyl such as cyclopropyl, cyclobutyl, cyclopentyl, cyclohexyl, cycloheptyl, cyclooctyl, bicyclo-[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo-[4.3.1]decyl, etc.) and, preferably, saturated C₃₋₆ alicyclic hydrocarbon groups.

The unsaturated alicyclic hydrocarbon group includes unsaturated C₅₋₁₂ alicyclic hydrocarbon groups (e.g. C₅₋₁₂ cycloalkenyl such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl, 2-cyclopenten-1-yl, 3-cyclopenten-1-yl, 2-cyclohexen-1-yl, 3-cyclohexen-1-yl, etc. and C₅₋₁₂ cycloalkadienyl groups such as 2,4-cyclopentadien-1-yl, 2,4-cyclohexadien-1-yl, 2,5-dicyclohexadien-1-yl, etc.).

The hydrocarbon group of said "hydrocarbon residue that may be substituted" for any of R¹, R² and R³ further includes saturated C₁₋₈ aliphatic hydrocarbon groups substituted by any of said saturated and unsaturated alicyclic hydrocarbon groups (such as C₃₋₇ cycloalkyl-C₁₋₈ alkyl and C₅₋₇ cycloalkenyl-C₁₋₈ alkyl, e.g. cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl, etc.).

The aryl group includes monocyclic and fused polycyclic C₆₋₁₄ aromatic carbocyclic groups. Among such aromatic carbocyclic groups are C₆₋₁₄ aryl groups such as phenyl, tolyl, xylyl, biphenyl, 1- or 2-naphthyl, 1-, 2- or 9-anthryl, 1-, 2-, 3-, 4-, or 9-phenanthryl, 1-, 2-, 4-, 5-, or 6-azulenyl, acenaphthylenyl, etc. and, among them, C₆₋₁₀ aryl groups such as phenyl, 1-naphthyl and 2-naphthyl are preferred.

The hydrocarbon group of said "hydrocarbon residue that may be substituted" for any of R¹, R², and R³ may have 1-3 optional substituent groups in substitutable positions. Among such substituent groups may be mentioned aryl that may be substituted, cycloalkyl and cycloalkenyl groups which may be substituted, heterocyclic groups that may be substituted, carboxy that may be esterified, carbamoyl that may be substituted, amino that may be substituted, hydroxy that may be substituted, thiol that may be substituted, halogen (e.g. fluorine, chlorine, bromine, iodine), and phosphono that may be substituted.

The aryl group of said aryl that may be substituted includes C₆₋₁₄ aryl groups such as phenyl, naphthyl, anthryl, phenanthryl, acenaphthylenyl, etc. and is preferably phenyl, 1-naphthyl or 2-naphthyl. The aryl group may have 1-2 optional substituent groups in substitutable positions, which substituent group or groups may for example be hydroxy, alkoxy (e.g. C₁₋₃ alkoxy such as methoxy, ethoxy, propoxy, etc.) that may be substituted, halogen (e.g. fluorine, chlorine, bromine and iodine), and alkyl (e.g. C₁₋₃ alkyl such as methyl, ethyl, propyl, etc.) that may be substituted. The alkoxy and alkyl mentioned above may each have 1-2 optional substituent groups such as phosphono that may be substituted (e.g. phosphoryl, dimethoxyphosphoryl, diethoxyphosphoryl, etc.), in substitutable positions.

The cycloalkyl group of said cycloalkyl that may be substituted includes C₃₋₇ cycloalkyl groups such as cyclopropyl, cyclobutyl, cyclopentyl, cyclohexyl, and cycloheptyl, among others. The substituent group or groups on said cycloalkyl that may be substituted may be similar in kind and number to the substituent group or groups mentioned for said aryl that may be substituted.

The cycloalkenyl group of said cycloalkenyl that may be substituted includes C₃₋₆ cycloalkenyl groups such as cyclopropenyl, cyclobutenyl, cyclopentenyl, and cyclohexenyl, among others. The substituent group or groups on said cycloalkenyl that may be substituted may be similar in kind and number to the substituent group or groups mentioned for said aryl that may be substituted.

The heterocyclic group of said heterocyclic group that may be substituted includes heteroaromatic and saturated or unsaturated non-aromatic heterocyclic (heteroaliphatic) groups each containing at least 1 hetero-atom selected from among oxygen, sulfur and nitrogen but is preferably an heteroaromatic group.

The "heterocyclic group that may be substituted" is exemplified by aromatic heterocyclic groups having at least 1 hetero atom selected from atoms of oxygen, sulfur and nitrogen as a ring-constituting atom (ring atom), and saturated or unsaturated non-aromatic heterocyclic groups (aliphatic heterocyclic groups), with preference given to aromatic heterocyclic groups. The aromatic heterocyclic group is exemplified by 5- to 7-membered aromatic heterocyclic groups containing 1 atom of sulfur, nitrogen or oxygen, 5- to 6-membered aromatic heterocyclic groups containing 2 to 4 atoms of nitrogen and 5- or 6-membered aromatic heterocyclic groups containing 1 or 2 atoms of nitrogen and 1 atom of sulfur or oxygen. These aromatic heterocyclic groups may have condensed with a 6-membered ring containing 2 or fewer atoms of nitrogen, a benzene ring or a 5-membered ring containing 1 atom of sulfur. The heteroaromatic group includes monocyclic heteroaromatic groups (e.g. furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.) and fused heteroaromatic groups (e.g. benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.). Particularly preferred are furyl, thienyl, indolyl, isoindolyl, pyrazinyl, pyridyl and pyrimidinyl. The non-aromatic heterocyclic group is exemplified by 5- to 7- membered non-aromatic heterocyclic groups containing 1 atom of sulfur, nitrogen or oxygen, and 4- to 7-membered non-aromatic heterocyclic groups containing 1 atom of nitrogen and 3 or fewer atoms selected from nitrogen, oxygen and sulfur. The non-aromatic heterocyclic group includes oxiranyl, azetidinyl, oxetanyl, thietanyl, pyrrolidinyl, tetrahydrofuryl, thiolanyl, piperidyl, tetrahydropyranyl, morpholinyl, thiomorpholinyl and piperazinyl, among others. Substituent groups for said heterocyclic group that may be substituted may for example be C₁₋₃ alkyl groups (e.g. methyl ethyl, propyl, etc.).

The carboxy that may be esterified includes carboxy, (C₁₋₆ alkoxy)carbonyl groups (e.g. methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl, tert-pentyloxycarbonyl, etc.), (C₆₋₁₀ aryloxy)carbonyl groups (e.g. phenoxycarbonyl, 1-naphthoxycarbonyl, etc.), and (C₇₋₁₃ aralkyloxy)carbonyl groups (e.g. benzyloxycarbonyl), among others. Particularly preferred are carboxy, methoxycarbonyl and ethoxycarbonyl.

Substituent groups for said carbamoyl that may be substituted may for example be lower(C₁₋₆)alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₃ aralkyl (e.g. benzyl, phenethyl, etc.). One or two, which may be the same or different, of such substituent groups may be present in substitutable positions.

Substituent groups for said amino that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₃ aralkyl (e.g. benzyl, phenethyl, etc.). One or two, which may be the same or different, of such substituent groups may be present.

Substituent groups for said hydroxy that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₃ aralkyl (e.g. benzyl, phenethyl, etc.).

Substituent groups for said thiol that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₃ aralkyl (e.g. benzyl, phenethyl, etc.).

The substituent for said phosphono group that may be substituted is exemplified by lower (C₁₋₆) alkyls (e.g., methyl, ethyl, propyl, isopropyl, butyl, isobutyl, sec-butyl, tert-butyl, pentyl, isopentyl, neopentyl, hexyl, isohexyl), lower (C₁₋₆) alkoxys (e.g., methoxy, ethoxy, propoxy, isopropoxy, butoxy, isobutoxy, sec-butoxy, tert-butoxy, pentyloxy, isopentyloxy, neopentyloxy, hexyloxy, isohexyloxy). Said phosphono groups include phosphoryl, dimethoxyphosphoryl, diethoxyphosphoryl, dipropoxyphosphoryl, diisopropoxyphosphoryl, ethylenedioxyphosphoryl, trimethylenedioxyphosphoryl and tertramethylenedioxyphosphoryl.

When the hydrocarbon moiety of said "hydrocarbon group that may be substituted" for any of R¹, R² and R³ is an alicyclic hydrocarbon group or an aryl group, the substituent group or groups may be aliphatic hydrocarbon groups that may be substituted. Among such aliphatic hydrocarbon groups are those saturated and unsaturated (preferably saturated) hydrocarbon groups which have been mentioned for the "hydrocarbon group that may be substituted" for any of R¹, R² and R³ and is preferably alkyl (e.g. C₁₋₃ alkyl such as methyl, ethyl, propyl, etc.). Such aliphatic hydrocarbon group may have 1-2 substituent groups in substitutable positions, which substituent group or groups may for example be phosphono that may be substituted (e.g. phosphoryl, dimethoxyphosphoryl, diethoxyphosphoryl, etc.).

In particular, the hydrocarbon group of said "hydrocarbon residue that may be substituted" for R¹, R² and R³ is preferably alkyl, more preferably C₁₋₁₀ alkyl, and for still better results, straight-chain or branched lower(C₁₋₆) alkyl (e.g. methyl, ethyl, propyl, isopropyl, butyl, isobutyl, sec-butyl, tert-butyl, pentyl, isopentyl, neopentyl, tert-pentyl, hexyl, isohexyl, 4-methylpentyl, 1,1-dimethylbutyl, 2,2-dimethylbutyl, 3,3-dimethylbutyl, 2-ethylbutyl, etc.). The preferred substituent group or groups for said "hydrocarbon group that may be substituted" are aryl (preferably phenyl) that may be substituted and heterocyclic groups that may be substituted.

More preferably, the "hydrocarbon residue that may be substituted" as represented by any of R¹, R² and R³ is alkyl that may be substituted by an aryl or heterocyclic ring (preferably heterocyclic ring). Referring to this alkyl (preferably lower(C₁₋₆) alkyl and more preferably C₁₋₄ alkyl) that may be substituted by an aryl or heterocyclic ring, the alkyl group substituted by an aryl group (aryl-substituted alkyl) includes various groups formed between a C₆₋₁₄ monocyclic or fused polycyclic aromatic hydrocarbon group (e.g. phenyl, naphthyl, anthryl, phenanthryl, acenaphthylenyl, etc.) and a C₁₋₆ alkyl group (for example, benzyl, 2-phenylethyl, 3-phenylpropyl, 2-phenylpropyl, 1-phenylpropyl, α-naphthylmethyl, α-naphthylethyl, β-naphthylmethyl, β-naphthylethyl, etc.), the heterocycle-substituted alkyl includes various groups formed between a heteroaromatic group and a C₁₋₆ alkyl group (preferably C₁₋₄ alkyl), where the heteroaromatic group includes but is not limited to 2-furyl, 3-furyl, 2-thienyl, 3-thienyl, 2-pyridyl, 3-pyridyl, 4-pyridyl, 2-pyrimidinyl, 4-pyrimidinyl, 5-pyrimidinyl, 6-pyrimidinyl, 3-pyridazinyl, 4-pyridazinyl, 2-pyrazinyl, 2-pyrrolyl, 3-pyrrolyl, 2-imidazolyl, 4-imidazolyl, 5-imidazolyl, 3-pyrazolyl, 4-pyrazolyl, isothiazolyl, isoxazolyl, 2-thiazolyl, 4-thiazolyl, 5-thiazolyl, 2-oxazolyl, 4-oxazolyl, 5-oxazolyl, 1,2,4-triazol-3-yl, 1,2,3-triazol-4-yl, tetrazol-5-yl, benzimidazol-2-yl, indol-2-yl, indol-3-yl, 1H-indazolyl, benzo[b]furanyl, isobenzofuranyl, benzo[b]thienyl, 1H-pyrrolo[2,3-b]pyrazin-2-yl, 1H-pyrrolo[2,3-b]pyridin-6-yl, 1H-imidazo[4,5-b]pyridin-2-yl, 1H-imidazo[4,5-c]pyridin-2-yl, 1H-imidazo[4,5-b]pyrazin-2-yl, and so on. Preferred are 2-pyridyl, 3-pyridyl, 4-pyridyl, 4-imidazolyl, 2-thienyl, 2-furyl, indol-2-yl and indol-3-yl, among others.

R¹ preferably represents heterocycle-substituted alkyl and, for still better results, indol-3-ylmethyl.

R² and R³ may be the same or different and each preferably represents a straight-chain or branched C₁₋₆ alkyl and, for still better results, sec-butyl, benzyl, isobutyl, or isopropyl.

The preferred combination of R¹ and R² is that R¹ represents indol-3-ylmethyl and R² represents sec-butyl, benzyl, isobutyl or isopropyl.

The preferred combination of R¹, R² and R³ is that R¹ represents indol-3-ylmethyl, R² represents sec-butyl, benzyl, isobutyl or isopropyl, and R³ represents sec-butyl, benzyl, isobutyl or isopropyl.

Referring to R⁴ in the formulas (I) and (II), said "acyl" includes acyl groups derived from organic acids which may be substituted. Among such acyl groups derived from organic acids and optionally substituted are acyl groups derived from carbamic acids which may be substituted, thiocarbamic acids which may be substituted, carboxylic acids which may be substituted, sulfinic acids which may be substituted, and sulfonic acids which may be substituted. Thus included are groups which can be represented by the formula -CONHR⁷, -CSNHR⁸, -COR⁹, -SOR¹⁰, or -SO₂R¹¹ [where R⁷, R⁸, R⁹, R¹⁰ and R¹¹ each represents hydrogen or a hydrocarbon or heterocyclic residue that may be substituted].

The "hydrocarbon residue that may be substituted" for any of R⁷, R⁸, R⁹, R¹⁰ and R¹¹ may be the same hydrocarbon residue as the "substituted hydrocarbon residue" for R¹, R² and R³.

The hydrocarbon group of said "substituted hydrocarbon residue that may be substituted" for any of R⁷, R⁸, R⁹, R¹⁰ and R¹¹ may have 1-3 optional substituents in substitutable positions and such substituents may be similar to those substituents mentioned for the "hydrocarbon residue that may be substituted" for R¹, R² and R³.

The heterocyclic group of said "heterocyclic residue that may be substituted" for any of R⁷, R⁸, R⁹, R¹⁰ and R¹¹ may be a heterocyclic group similar to that of the "heterocyclic residue that may be substituted" mentioned as a substituent for R¹, R² and R³.

This heterocyclic group of the "heterocyclic residue that may be substituted" for R⁷, R⁸, R⁹, R¹⁰ and R¹¹ may have 1-3 optional substituents in substitutable positions and such substituents may be similar to those mentioned for the "hydrocarbon residue that may be substituted" for R¹, R² and R³.

The acyl for R⁴ includes but is not limited to aliphatic acyl groups such as alkanoyl (e.g. lower C₁₋₆ alkyl-carbonyl groups such as formyl, acetyl, propionyl, butyryl, isobutyryl, valeryl, isovaleryl, pivaloyl, hexanoyl, valproyl, etc.), alkenoyl (e.g. (lower C₂₋₆ alkenyl)carbonyl groups such as acryloyl, methacryloyl, crotonoyl, isocrotonoyl, etc.), cycloalkanecarbonyl (e.g. (C₃₋₆ cycloalkyl)carbonyl groups such as cyclopropanecarbonyl, cyclobutanecarbonyl, cyclopentanecarbonyl, cyclohexanecarbonyl, etc.), cycloalkenylcarbonyl (e.g. (C₃₋₇ cycloalkenyl)carbonyl groups such as cyclopropenylcarbonyl, cyclobutenylcarbonyl, cyclopentenylcarbonyl, cyclohexenylcarbonyl, etc.), and alkanesulfonyl (e.g. (lower C₁₋₆ alkyl)sulfonyl groups such as mesyl, ethanesulfonyl, propanesulfonyl, etc.), and aromatic acyl groups such as aroyl (e.g. (C₆₋₁₀ aryl)carbonyl such as benzoyl, p-toluoyl, 1-naphthoyl, 2-naphthoyl, etc.), arylalkanoyl (e.g. C₆₋₁₀ aryl-substituted (C₁₋₆ alkyl)carbonyl such as phenylacetyl, phenylpropionyl, hydroatropoyl, phenylbutyryl, etc.), arylalkenoyl (e.g. C₆₋₁₀ aryl-substituted (C₂₋₆ alkenyl)carbonyl such as cinnamoyl, atropoyl, etc.), and C₆₋₁₀ arylsulfonyl (e.g. arylsulfonyl groups such as benzenesulfonyl, p-toluenesulfonyl, etc.), heteroaromatic ring-carbonyl (e.g. heteroaromatic ring-carbonyl groups such as furoyl, thenoyl, nicotinoyl, isonicotinoyl, pyrrolecarbonyl, oxazolecarbonyl, imidazolecarbonyl, pyrazolecarbonyl, etc.), and heteroaromatic alkanoyl (e.g. heteroaromatic ring-substituted (C₁₋₆ alkyl)carbonyl groups such as thienylacetyl, thienylpropanoyl, furylacetyl, thiazolylacetyl, 1,2,4-thiadiazolylacetyl, pyridylacetyl, etc.).

Among the above-mentioned acyl groups for R⁴, groups of the formula -COR⁹ and groups of the formula - SO₂R¹¹ are preferred and groups of the formula -SO₂R¹¹ are still more preferred.

Preferred among groups of the formula -COR⁹ are those groups which can be represented by the formula - COR^{9'} [R^{9'} represents hydrogen or an alkyl, alkenyl or aromatic group that may be substituted].

The alkyl group of the "alkyl that may be substituted" for R^{9'} includes lower(C₁₋₆) alkyl (e.g. methyl, ethyl, propyl, isopropyl, butyl, isobutyl, sec-butyl, tert-butyl, pentyl, isopentyl, neopentyl, tert-pentyl, hexyl, isohexyl, etc.).

The alkenyl group of the "alkenyl that may be substituted" for R^{9'} includes lower(C₂₋₆) alkenyl (e.g. ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, etc.).

The alkyl group of the "alkyl that may be substituted" and the alkenyl group of the "alkenyl that may be substituted", both for R^{9'}, may each have 1-2 optional substituent groups in substitutable positions, which substituent group or groups may be aryl (preferably phenyl, 1-naphthyl and 2-naphthyl) that may be substituted. The aryl mentioned above may have 1-2 optional substituent groups in substitutable positions, which substituent group or groups may for example be alkyl (e.g. C₁₋₃ alkyl such as methyl, ethyl, propyl, etc.) that may be substituted. The alkyl mentioned above may have 1-2 optional substituent groups in substitutable positions, which substituent group or groups may for example be optionally substituted phosphono groups (e.g. phosphoryl, dimethoxyphosphoryl, diethoxyphosphoryl, etc.).

The preferred aromatic group of the "aromatic group that may be substituted" for R^{9'} includes furyl, thienyl, indolyl, isoindolyl, pyrazinyl, pyridyl and pyrimidinyl, among others.

This aromatic group of the "aromatic group that may be substituted" for R^{9'} may have optional substituents in substitutable positions, which substituent group or groups may for example be C₁₋₃ alkyl (e.g. methyl, ethyl, propyl, etc.) and/or halogen.

The group of the formula -SO₂R¹¹ is more preferably a group of the formula -SO₂R^{11'} [R^{11'} represents aryl that may be substituted].

The aryl group of said "aryl that may be

substituted" for R^{11'} may for example be C₆₋₁₄ aryl such as phenyl, 1-naphthyl or 2-naphthyl. This aryl group may have 1-2 optional substituent groups in substitutable positions, which substituent group or groups may for example be alkyl (e.g. C₁₋₃ alkyl such as methyl, ethyl, propyl, etc.) that may be substituted. This alkyl, in turn, may have 1-2 optional substituent groups in substitutable positions, which substituent group or groups may for example be phosphono that may be substituted (e.g. phosphoryl, dimethoxyphosphoryl, diethoxyphosphoryl, etc.).

Referring to the above formula (I), the hydrocarbon group of said "hydrocarbon residue that may be substituted" for R⁵ and R⁶ includes saturated and unsaturated aliphatic acyclic hydrocarbon groups, saturated and unsaturated alicyclic hydrocarbon groups, and aryl groups.

The saturated aliphatic hydrocarbon group includes but is not limited to straight-chain or branched C₁₋₁₀ saturated aliphatic hydrocarbon groups (e.g. C₁₋₁₀ alkyl groups such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.) and is preferably a straight-chain or branched C₁₋₆ saturated aliphatic hydrocarbon group.

The unsaturated aliphatic hydrocarbon group includes straight-chain and branched C₂₋₁₀ unsaturated aliphatic hydrocarbon groups (e.g. C₂₋₁₀ alkenyl such as ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, etc. and C₂₋₁₀ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 2,4-hexadiynyl, 5-hexynyl, 1-heptynyl, 1-octynyl, etc.) and, preferably, straight-chain and branched C₂₋₆ unsaturated aliphatic hydrocarbon groups.

The saturated alicyclic hydrocarbon group includes saturated C₃₋₁₂ alicyclic hydrocarbon groups (e.g. C₃₋₁₂ cycloalkyl such as cyclopropyl, cyclobutyl, cyclopentyl, cyclohexyl, cycloheptyl, cyclooctyl, bicyclo-[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo-[4.3.1]decyl, etc.) and, preferably, saturated C₃₋₆ alicyclic hydrocarbon groups.

The unsaturated alicyclic hydrocarbon group includes unsaturated C₅₋₁₂ alicyclic hydrocarbon groups (e.g. C₅₋₁₂ cycloalkenyl such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl, 2-cyclopenten-1-yl, 3-cyclopenten-1-yl, 2-cyclohexen-1-yl, 3-cyclohexen-1-yl, etc. and C₅₋₁₂ cycloalkadienyl groups such as 2,4-cyclopentadien-1-yl, 2,4-cyclohexadien-1-yl, 2,5-cyclohexadien-1-yl, etc.).

The hydrocarbon group of said "hydrocarbon residue that may be substituted" for R⁵ and R⁶ further includes saturated C₁₋₈ aliphatic hydrocarbon groups substituted by any of said saturated and unsaturated alicyclic hydrocarbon groups (such as C₃₋₇ cycloalkyl-C₁₋₈ alkyl and C₅₋₇ cycloalkenyl-C₁₋₈ alkyl, e.g. cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl, etc.).

The aryl group includes monocyclic and fused polycyclic C₆₋₁₄ aromatic carbocyclic groups. Among such aromatic carbocyclic groups are C₆₋₁₄ aryl groups such as phenyl, tolyl, xylyl, biphenyl, 1- or 2-naphthyl, 1-, 2- or 9-anthryl, 1-, 2-, 3-, 4-, or 9-phenanthryl, 1-, 2-, 4-, 5-, or 6-azulenyl, acenaphthylenyl, etc. and, among them, C₆₋₁₀ aryl groups such as phenyl, 1-naphthyl and 2-naphthyl are preferred.

The hydrocarbon moiety of said "hydrocarbon group that may be substituted" for R⁵, and R⁶ may have 1-3 optional substituent groups in substitutable positions. Among such substituent groups may be mentioned aryl that may be substituted, cycloalkyl and cycloalkenyl groups which may be substituted, heterocyclic groups that may be substituted, carboxy that may be esterified, carbamoyl that may be substituted, amino that may be substituted, hydroxy that may be substituted, thiol that may be substituted, halogen (e.g. fluorine, chlorine, bromine, iodine), and phosphono that may be substituted.

The aryl group of said aryl that may be substituted includes C₆₋₁₄ aryl groups such as phenyl, naphthyl, anthryl, phenanthryl, acenaphthylenyl, etc. and is preferably phenyl, 1-naphthyl or 2-naphthyl. The aryl group may have 1-2 optional substituent groups in substitutable positions, which substituent group or groups may for example be hydroxy, alkoxy (e.g. C₁₋₃ alkoxy such as methoxy, ethoxy, propoxy, etc.) that may be substituted, halogen (e.g. fluorine, chlorine, bromine and iodine), and alkyl (e.g. C₁₋₃ alkyl such as methyl, ethyl, propyl, etc.) that may be substituted. The alkoxy and alkyl mentioned above may each have 1-2 optional substituent groups such as phosphono that may be substituted (e.g. phosphoryl, dimethoxyphosphoryl, diethoxyphosphoryl, etc.), in substitutable positions.

The cycloalkyl group of said cycloalkyl that may be substituted includes C₃₋₇ cycloalkyl groups such as cyclopropyl, cyclobutyl, cyclopentyl, cyclohexyl, and cycloheptyl, among others. The substituent group or groups on said cycloalkyl that may be substituted may be similar in kind and number to the substituent group or groups mentioned for said aryl that may be substituted.

The cycloalkenyl group of said cycloalkenyl that may be substituted includes C₃₋₆ cycloalkenyl groups such as cyclopropenyl, cyclobutenyl, cyclopentenyl, and cyclohexenyl, among others. The substituent group or groups on said cycloalkenyl that may be substituted may be similar in kind and number to the substituent group or groups mentioned for said aryl that may be substituted.

The heterocyclic group of said heterocyclic group that may be substituted includes heteroaromatic and saturated or unsaturated non-aromatic heterocyclic (heteroaliphatic) groups each containing at least 1 hetero-atom selected from among oxygen, sulfur and nitrogen but is preferably an heteroaromatic group.

The "heterocyclic group that may be substituted" is exemplified by aromatic heterocyclic groups having at least 1 hetero atom selected from atoms of oxygen, sulfur and nitrogen as a ring-constituting atom (ring atom), and saturated or unsaturated non-aromatic heterocyclic gorups (aliphatic heterocyclic groups), with preference given to aromatic heterocyclic groups. The aromatic heterocyclic group is exemplified by 5- to 7-membered aromatic heterocyclic groups containing 1 atom of sulfur, nitrogen or oxygen, 5- to 6-membered aromatic heterocyclic groups containing 2 to 4 atoms of nitrogen and 5- or 6-membered aromatic heterocyclic groups containing 1 or 2 atoms of nitrogen and 1 atom of sulfur or oxygen. These aromatic heterocyclic groups may have condensed with a 6-membered ring containing 2 or fewer atoms of nitrogen, a benzene ring or a 5-membered ring containing 1 atom of sulfur. The heteroaromatic group includes monocyclic heteroaromatic groups (e.g. furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.) and fused heteroaromatic groups (e.g. benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.). Particularly preferred are furyl, thienyl, indolyl, isoindolyl, pyrazinyl, pyridyl and pyrimidinyl. The non-aromatic heterocyclic group is exemplified by 5- to 7-membered non-aromatic heterocyclic groups containing 1 atom of sulfur, nitrogen or oxygen, and 4- to 7-membered non-aromatic heterocyclic groups containing 1 atom of nitrogen and 3 or fewer atoms selected from nitrogen, oxygen and sulfur. The non-aromatic heterocyclic group includes oxiranyl, azetidinyl, oxetanyl, thietanyl, pyrrolidinyl, tetrahydrofuryl, thiolanyl, piperidyl, tetrahydropyranyl, morpholinyl, thiomorpholinyl and piperazinyl, among others. Substituent groups for said heterocyclic group that may be substituted may for example be C₁₋₃ alkyl groups (e.g. methyl ethyl, propyl, etc.).

The carboxy that may be esterified includes carboxy, (C₁₋₆ alkoxy)carbonyl groups (e.g. methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl, tert-pentyloxycarbonyl, etc.), (C₆₋₁₀ aryloxy)carbonyl groups (e.g. phenoxycarbonyl, 1-naphthoxycarbonyl, etc.), and (C₇₋₁₃ aralkyloxy)carbonyl groups (e.g. benzyloxycarbonyl), among others. Particularly preferred are carboxy, methoxycarbonyl and ethoxycarbonyl.

Substituent groups for said carbamoyl that may be substituted may for example be lower(C₁₋₆)alkyl (e.g. methyl, ethyl, propyl, isopropyl, butyl, isobutyl, sec-butyl, tert-butyl, pentyl, isopentyl, neopentyl, hexyl, isohexyl, etc.) that may be substituted, C₃₋₆ cycloalkyl (e.g. cyclopropyl, cyclobutyl, cyclopentyl, cyclohexyl, etc.) that may be substituted, C₆₋₁₀ aryl (e.g. phenyl, 1-naphthyl, 2-naphthyl, etc.) that may be substituted, and C₇₋₁₃ aralkyl (e.g. benzyl, phenethyl, etc.) that may be substituted. One or two, which may be the same or different, of such substituent groups may be present in substitutable positions. The substituent group or groups for said lower(C₁₋₆)alkyl that may be substituted and said C₃-₆ cycloalkyl that may be substituted may for example be carboxy, heteroaromatic groups (e.g. furyl, thienyl, indolyl, isoindolyl, pyrazinyl, pyridyl, pyrimidyl, imidazolyl, etc.), amino, hydroxy, and phenyl, among others. The substituent group or groups for said aryl that may be substituted and said aralkyl that may be substituted may for example be halogen (e.g. fluorine, chlorine, bromine, iodine) and carboxy. Two substituents on the nitrogen atom may, taken together with the nitrogen atom, form a cyclic amino group such as 1-azetidinyl, 1-pyrrolidinyl, piperidino, morpholino, 1-piperazinyl or the like.

Substituent groups for said amino that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.) that may be substituted, C₃₋₆ cycloalkyl (e.g. cyclopropyl, cyclobutyl, cyclopentyl, cyclohexyl, etc.) that may be substituted, C₆₋₁₀ aryl (e.g. phenyl, 1-naphthyl, 2-naphthyl, etc.) that may be substituted, and C₇₋₁₃ aralkyl (e.g. benzyl, phenethyl, etc.) that may be substituted. One or two, which may be the same or different, of such substituent groups may be present. The substituent group or groups for said lower(C₁₋₆)alkyl that may be substituted and said C₃₋₆ cycloalkyl that may be substituted may for example be carboxy, heteroaromatic groups (e.g. furyl, thienyl, indolyl, isoindolyl, pyrazinyl, pyridyl, pyrimidyl, imidazolyl, etc.), amino, hydroxy, and phenyl, among others. The substituent group or groups for said aryl that may be substituted and said aralkyl that may be substituted may for example be halogen (e.g. fluorine, chlorine, bromine, iodine) and carboxy. Furthermore, two substituents on the nitrogen atom may, taken together with the nitrogen atom, form a cyclic amino group such as 1-azetidinyl, 1-pyrrolidinyl, piperidino, morpholino, 1-piperazinyl or the like.

Substituent groups for said hydroxy that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₃ aralkyl (e.g. benzyl, phenethyl, etc.).

Substituent groups for said thiol that may be substituted may for example be lower(C₁₋₆) alkyl (e.g. methyl, ethyl, propyl, isopropyl, butyl, isobutyl, sec-butyl, tert-butyl, pentyl, isopentyl, neopentyl, hexyl, isohexyl, etc.), C₃₋₆ cycloalkyl (e.g. cyclopropyl, cyclobutyl, cyclopentyl, cyclohexyl, etc.), C₆₋₁₀ aryl, (e.g. phenyl, 1-naphthyl, 2-naphthyl, etc.), and C₇₋₁₃ aralkyl (e.g. benzyl, phenethyl, etc.).

The substituent for said phosphono group that may be substituted is exemplified by lower (C₁₋₆) alkyls (e.g., methyl, ethyl, propyl, isopropyl, butyl, isobutyl, sec-butyl, tert-butyl, pentyl, isopentyl, neopentyl, hexyl, isohexyl), lower (C₁₋₆) alkoxys (e.g., methoxy, ethoxy, propoxy, isopropoxy, butoxy, isobutoxy, sec-butoxy, tert-butoxy, pentyloxy, isopentyloxy, neopentyloxy, hexyloxy, isohexyloxy). Said phosphons groups include phosphoryl, dimethoxyphosphoryl, diethoxyphosphoryl, dipropoxyphosphoryl, diisopropoxyphosphoryl, ethylenedioxyphosphoryl, trimethylenedioxyphosphoryl and tetramethylenedioxyphosphoryl.

When the hydrocarbon group of said "hydrocarbon residue that may be substituted" for R⁵ and R⁶ is an alicyclic hydrocarbon group or an aryl group, the substituent group or groups may be aliphatic hydrocarbon groups that may be substituted. Among such aliphatic hydrocarbon groups are those saturated and unsaturated (preferably saturated) hydrocarbon groups which have been mentioned for the "hydrocarbon group that may be substituted" for R⁵ and R⁶ and is preferably alkyl (e.g. C₁₋₃ alkyl such as methyl, ethyl, propyl, etc.). Such aliphatic hydrocarbon group may have 1-2 substituent groups in substitutable positions, which substituent group or groups may for example be phosphono that may be substituted (e.g. phosphoryl, dimethoxyphosphoryl, diethoxyphosphoryl, etc.).

In particular, the hydrocarbon group of said "hydrocarbon residue that may be substituted" for R⁵ and R⁶ is preferably alkyl, more preferably C₁₋₁₀ alkyl, and for still better results, straight-chain or branched lower(C₁₋₆) alkyl (e.g. methyl, ethyl, propyl, isopropyl, butyl, isobutyl, sec-butyl, tert-butyl, pentyl, isopentyl, neopentyl, tert-pentyl, hexyl, isohexyl, 4-methylpentyl, 1,1-dimethylbutyl, 2,2-dimethylbutyl, 3,3-dimethylbutyl, 2-ethylbutyl, etc.).

R⁵ and R⁶ may be the same or different but are preferably the same.

Preferably, R⁵ and R⁶ each represents unsubstituted alkyl (preferably methyl or ethyl).

Referring to m and n in the formulas (I) and (II), it is preferable that i) m be equal to 1 and n be equal to 1, ii) m be equal to 1 and n be equal to 0, or iii) m be equal to 0 and n be equal to 0. The still preferred combination is that m is equal to 1 and n is equal to 0.

Where the formula (I) is represented by the formula (I') 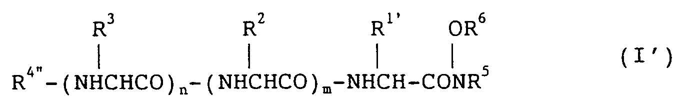 wherein R^{1'} represents indol-3-ylmethyl; R^{4''} represents carboxy that may be esterified or acyl; the other symbols have the same meanings as defined hereinbefore. Moreover, where the formula (II) is represented by the formula (II') 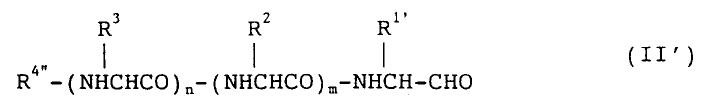 wherein R^{1'} represents indol-3-ylmethyl; R^{4''} represents carboxy that may be esterified or acyl; the other symbols have the same meanings as defined hereinbefore.

The "carboxy that may be esterified" includes groups of the formula -COOR¹² [R¹² typically represents hydrogen, C₁₋₆ alkyl, C₂₋₆ alkenyl or C₆₋₁₀ aralkyl]. Typical are the group formed between carboxy and C₁₋₆ alkyl, such as C₁₋₆ alkoxycarbonyl (e.g. methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.), the group formed between carboxy and C₂₋₆ alkenyl, such as C₂₋₆ alkenyloxycarbonyl (e.g. allyloxycarbonyl, crotyloxycarbonyl, 2-pentenyloxycarbonyl, 3-hexenyloxycarbonyl, etc.), and the group formed between carboxy and C₆₋₁₀ aralkyl, such as C₆₋₁₀ aralkyloxycarbonyl (e.g. benzyloxycarbonyl, phenethyloxycarbonyl, etc.).

The compound of the formula (II) that can be produced in accordance with the present invention includes but is not limited to the following species.
- N-(4-Toluenesulfonyl)-(L)-isoleucyl-(L)-tryptophanal
- N-(t-Butoxycarbonyl)-(L)-isoleucyl-(L)-tryptophanal
- N-(1-Naphthylsulfonyl)-(L)-isoleucyl-(L)-tryptophanal
- N-(1-Naphthylsulfonyl)-(L)-isoleucyl-(L)-isoleucyl-(L)-tryptophanal
- N-Benzylcarbamoyl-(L)-isoleucyl-(L)-tryptophanal
- N-[(2-Cyclohexylethyl)carbamoyl]-(L)-isoleucyl-(L)-tryptophanal
- N-(3-Trifluoromethylphenylcarbamoyl)-(L)-isoleucyl-(L)-tryptophanal
- N-(2-Propylpentanoyl)-(L)-tryptophanal
- N-Dibenzylacetyl-(L)-tryptophanal
- N-Dibenzylacetyl-(L)-phenylalaninal
- N-(1-Naphthylsulfonyl)-(L)-isoleucyl-(L)-phenylalaninal
- N-(1-Naphthylsulfonyl)-(L)-isoleucyl-(L)-alaninal
- N-(2-Propylpentanoyl)-(L)-alanyl-(L)-tryptophanal
- N-(2-Propylpentanoyl)-(L)-valyl-(L)-tryptophanal

The salts of compounds of the formulas (I), (I'), (II) and (II') as falling under the purview of the present invention are preferably physiologically acceptable salts, thus including salts with inorganic bases, salts with organic bases, salts with inorganic acids, salts with organic acids, and salts with basic or acidic amino acids. Among preferred salts with inorganic bases are salts with alkali metals such as sodium, potassium, etc., salts with alkaline earth metals such as calcium, magnesium, etc., and aluminum salts. The preferred salts with organic bases are ammonium salts and salts with trimethylamine, triethylamine, pyridine, picoline, ethanolamine, diethanolamine, triethanolamine, dicyclohexylamine, N,N'-dibenzylethylenediamine, etc. The preferred salts with inorganic acids are salts with hydrochloric acid, hydrobromic acid, nitric acid, sulfuric acid, phosphoric acid, etc. The preferred salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. The preferred salts with basic amino acids are salts with arginine, lysine, ornithine, etc. The preferred salts with acidic amino acids are salts with aspartic acid, glutamic acid, etc.

Processes for producing compound (II) are now described. 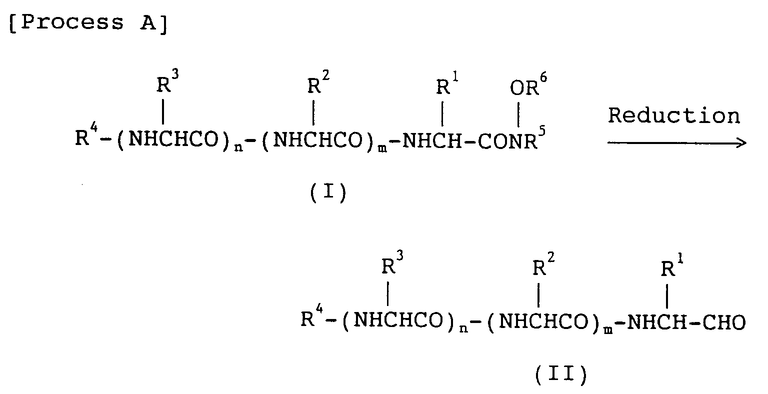 wherein each symbol has the same meaning as defined hereinbefore.

This reduction reaction is carried out using an aluminum hydride type reducing agent in accordance with the disclosure in Synthesis, p. 676 (1983) or Journal of Medicinal Chemistry 33, 11-13 (1990). The preferred reducing agent includes aluminum hydrides (e.g. diisobutylaluminum hydride, lithium aluminum hydride, sodium bis(2-methoxyethoxy)aluminum hydride, etc.). The amount of the reducing agent relative to the compound of the formula (I) is about 3-10 molar equivalents and preferably about 4-5 molar equivalents. The reaction time is about 10 minutes to 24 hours and preferably about 30 minutes to 5 hours. The reaction temperature is about -100°C to 100°C and preferably about -70°C to 60°C. The reaction solvent that can be conveniently employed includes aromatic hydrocarbons such as benzene, toluene, xylene, etc., halogenated hydrocarbons such as dichloromethane, chloroform, 1,2-dichloroethane, etc., and ethers such as diethyl ether, tetrahydrofuran, dioxane and so on. These solvents can be used singly or, if necessary, in a combination of two or more species.

The aldehyde derivative (II) thus obtained can be isolated and purified by the known purification procedures such as concentration, concentration in vacuo, solvent extraction, crystallization, recrystallization, redistribution, and chromatography, among other techniques.

The starting material for process A can be produced by the following and other processes. 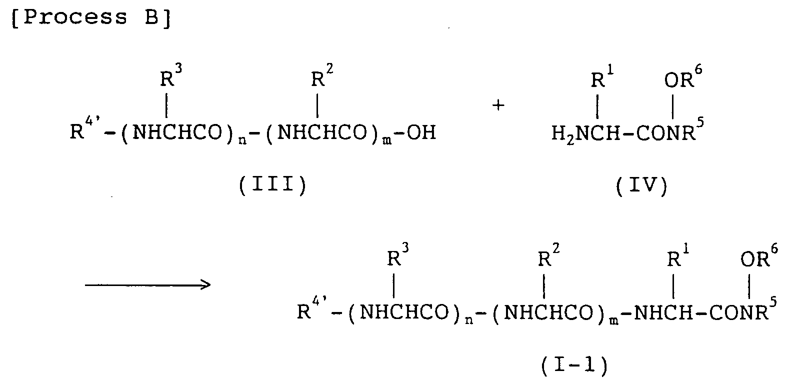 In the above reaction scheme, R^{4'} represents an acyl group derived from a carboxylic acid that may be substituted or a sulfonic acid that may be substituted; the other symbols have the same meanings as defined hereinbefore. However, where R¹ represents indol-3-ylmethyl, R^{4'} may be a carboxyl group that may be esterified.

According to this process, compound (III) or a reactive derivative of the carboxyl function thereof or a salt thereof is reacted with compound (IV) or a reactive derivative of the amino function thereof or a salt thereof to give compound (I-1).

The preferred reactive derivative of the amino function of compound (IV) may for example be a Schiff base-form imino compound available on reaction of compound (IV) with a carbonyl compound such as an aldehyde or a ketone or the enamine-form tautomer thereof; a silyl derivative available on reaction of compound (IV) with a silyl compound such as bis(trimethylsilyl)acetamide, mono(trimethylsilyl)-acetamide, bis(trimethylsilyl)urea or the like; or a derivative available on reaction of compound (IV) with phosphorus trichloride or phosgene. For the preferred salt of said compound (IV) or a reactive derivative thereof, reference may be had to the acid addition salts mentioned for compound (I). For example, the salts include salts with inorganic acids, salts with organic acids. The preferred salts with inorganic acids are salts with hydrochloric acid, hydrobromic acid, nitric acid, sulfuric acid, phosphoric acid, etc. The preferred salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

The preferred reactive derivative of the carboxyl function of compound (III) includes but is not limited to the acid halides, acid anhydrides, activated amides, and activated esters. An exemplary list of specific reactive derivatives comprises the corresponding acid chloride; acid azide; mixed acid anhydrides with substituted phosphoric acids, e.g. dialkylphosphoric acid, phenylphosphoric acid, diphenylphosphoric acid, dibenzylphosphoric acid, halophosphoric acids, etc., dialkyl phosphites, sulfurous acid, thiosulfuric acid, sulfuric acid, sulfonic acids, e.g. methanesulfonic acid, aliphatic carboxylic acids, e.g. acetic acid, propionic acid, butyric acid, isobutyric acid, pivalic acid, pentanoic acid, isopentanoic acid, trichloroacetic acid, etc., aromatic carboxylic acids, e.g. benzoic acid; symmetric acid anhydride; activated amides with imidazole, 4-substituted imidazole, dimethylpyrazole, triazole and tetrazole; activated esters such as cyanomethyl ester, methoxymethyl ester, dimethyliminomethyl ester, vinyl ester, propargyl ester, p-nitrophenyl ester, trichlorophenyl ester, pentachlorophenyl ester, mesylphenyl ester, phenylazophenyl ester, phenylthio ester, p-nitrophenyl ester, p-cresylthio ester, carboxymethylthio ester, pyranyl ester, pyridyl ester, piperidyl ester, 8-quinolylthio ester, etc., and esters with N-hydroxy compounds such as N,N-dimethylhydroxylamine, 1-hydroxy-2-(1H)-pyridone, N-hydroxysuccinimide, N-hydroxyphthalimide, 1-hydroxy-1H-benzotriazole, etc. These reactive derivatives can be selectively employed according to the species of compound (III). The preferred salt of the compound (III) or a reactive derivative thereof includes salts with bases, e.g. alkali metals such as sodium, potassium, etc. and alkaline earth metals such as calcium, magnesium, etc., ammonium salts, and salts with organic bases such as trimethylamine, triethylamine, pyridine, picoline, dicyclohexylamine, N,N-dibenzylethylenediamine and so on.

This reaction is generally carried out in water or the common solvent, e.g. alcohols such as methanol, ethanol, etc., acetone, dioxane, acetonitrile, chloroform, methylene chloride, ethylene chloride, tetrahydrofuran, ethyl acetate, N,N-dimethylformamide, pyridine and so on. However, the reaction can be conducted in any other solvent that does not adversely affect the reaction. Any of these common solvents can be used in admixture with water.

When compound (III) is used in the free acid form or in the form of a salt in this reaction, the reaction is preferably conducted in the presence of an ordinary condensing agent such as N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisopropylcarbodiimide, N-ethyl-N'-(3-dimethylaminopropyl)carbodiimide, N,N'-carbonylbis(2-methylimidazole), pentamethyleneketene-N-cyclohexylimine, diphenylketene-N-cyclohexylimine, ethoxyacetylene, 1-alkoxy-1-chloroethylene, trialkyl phosphites, ethyl polyphosphate, isopropyl polyphosphate, phosphorus oxychloride, diphenylphosphorylazide, thionyl chloride, oxalyl chloride, lower alkyl haloformates such as ethyl chloroformate, isopropyl chloroformate, etc., triphenylphosphine, 2-ethyl-7-hydroxybenzisoxazolium salt, 2-ethyl-5-(m-sulfophenyl)isoxazolium hydroxide internal salt, N-hydroxybenzotriazole, 1-(p-chlorobenzenesulfonyloxy)-6-chloro-1H-benzotriazole, and Vilsmeier reagents available on reaction of N,N'-dimethylformamide with thionyl chloride, phosgene, trichloromethyl chloroformate, phosphorus oxychloride, etc. This reaction may also be carried out in the presence of an inorganic base or an organic base, e.g. alkali metal hydrogen carbonates, tri(lower)alkylamines, pyridine, N-(lower)alkylmorpholines, N,N-di-(lower)alkylbenzylamines, etc. While the temperature is not critical, this reaction is usually carried out under cooling through warming, for example about -100°C to 100°C. The reaction time is about 30 minutes to 72 hours. 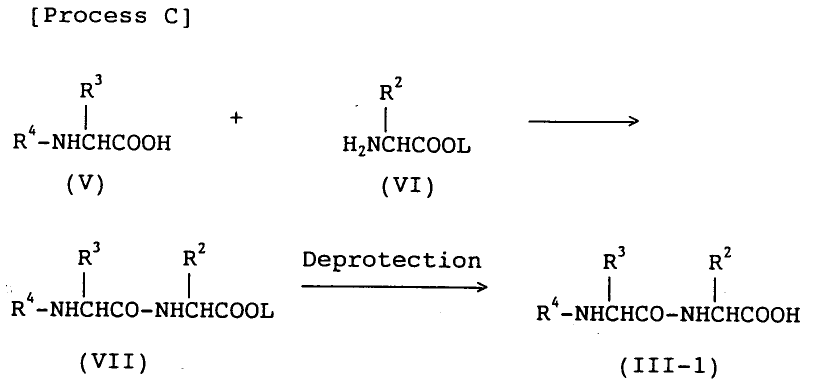 In the above reaction scheme, L represents a carboxy-protecting group; the other symbols have the same meanings as defined hereinbefore.

The carboxy-protecting group L includes a variety of protective groups in common usage in the field of peptide synthesis, for example ester residues.

In this process, R⁴ may be also a carboxyl group that may be esterified. The "carboxyl group that may be esterified" includes groups of the formula -COOR¹² (R¹² is the same definition as mentioned above).

In accordance with this process, compound (V) or a reactive derivative of the carboxyl function thereof or a salt thereof is reacted with compound (VI) or a reactive derivative of the amino function thereof or a salt thereof to produce compound (VII) and the carboxy-protecting group is then eliminated from compound (VII) to give compound (III-1). This reaction between compound (V) or a reactive derivative of the carboxyl function thereof or a salt thereof and compound (VI) or a reactive derivative of the amino function thereof or a salt thereof is carried out in the same manner as Process B.

Elimination of the carboxy-protecting group from compound (VII) can be carried out by any of the reactions used generally for removal of a carboxy-protecting group, for example hydrolysis, reduction, and deprotection with a Lewis acid. Where the carboxy-protecting group is an ester residue, it can be eliminated by hydrolysis or the deprotection reaction with a Lewis acid. The hydrolysis reaction is preferably carried out in the presence of a base or an acid. The preferred base includes a variety of inorganic bases such as alkali metal hydroxides (e.g. sodium hydroxide, potassium hydroxide, etc.), alkaline earth metal hydroxides (e.g. magnesium hydroxide, calcium hydroxide, etc.), alkali metal carbonates (e.g. sodium carbonate, potassium carbonate, etc.), alkaline earth metal carbonates (e.g. magnesium carbonate, calcium carbonate, etc.), alkali metal hydrogen carbonates (e.g. sodium hydrogen carbonate, potassium hydrogen carbonate, etc.), alkali metal acetates (e.g. sodium acetate, potassium acetate, etc.), alkaline earth metal phosphates (e.g. magnesium phosphate, calcium phosphate, etc.), alkali metal hydrogen phosphates (e.g. disodium hydrogen phosphate, dipotassium hydrogen phosphate, etc.), etc. and a variety of organic bases such as trialkylamines (e.g. trimethylamine, triethylamine, etc.), picoline, N-methylpyrrolidine, N-methylmorpholine, 1,5-diazabicyclo[4,3,0]non-5-ene, 1,4-diazabicyclo[2,2,2]non-5-ene, 1,8-diazabicyclo[5,4,0]-7-undecene, etc. The hydrolysis reaction using a base is carried out in water, a hydrophilic organic solvent, or a mixture thereof in many instances. The preferred acid is an organic or inorganic acid (e.g. formic acid, hydrochloric acid, sulfuric acid and so on). This hydrolysis reaction is generally carried out in water or the common solvent, e.g. alcohols such as methanol, ethanol, etc., acetone, dioxane, acetonitrile, chloroform, methylene chloride, ethylene chloride, tetrahydrofuran, ethyl acetate, N,N-dimethylformamide, pyridine and so on. However, the reaction can be conducted in any other solvent that does not adversely affect the reaction. Any of these common solvents can be used in admixture with water. The reaction temperature is not critical and can be liberally selected according to the species of carboxy-protecting group and the method of deprotection, for example about -100°C to 100°C. The reaction time is about 30 minutes to 24 hours.

The deprotection using a Lewis acid is carried out by reacting compound (VII) or a salt thereof with a Lewis acid such as boron trihalides (e.g. boron trichloride, boron trifluoride, etc.), titanium tetrahalides (e.g. titanium tetrachloride, titanium tetrabromide, etc.), aluminum halides (e.g. aluminum chloride, aluminum bromide, etc.), and trihaloacetic acids (e.g. trichloroacetic acid, trifluoroacetic acid, etc.). This deprotection reaction is preferably conducted in the presence of a cation acceptor (e.g anisole, phenol, etc.) and usually in a solvent such as nitroalkanes (e.g. nitromethane, nitroethane, etc.), alkylene halides (e.g. methylene chloride, ethylene chloride, etc.), diethyl ether, carbon disulfide, and other solvents which do not adversely affect the reaction. These solvents can be used as a suitable mixture. The reaction time is about 30 minutes to 48 hours. The reaction temperature is about -100°C to 100°C.

The reductive deprotection reaction is preferably applied to the deprotection of haloalkyl (e.g. 2-iodoethyl, 2,2,2-trichloroethyl, etc.) esters and aralkyl (e.g. benzyl) esters. The reduction method that can be employed includes but is not limited to the use of either a metal (e.g. zinc, zinc amalgam, etc.) or a chromium salt (e.g. chromous chloride, chromous acetate, etc.) and an organic or inorganic acid (e.g. acetic acid, propionic acid, hydrochloric acid, etc.) and the ordinary catalytic reduction with the aid of a routine metal catalyst (e.g. palladium-on-carbon, Raney nickel, etc.). The reaction temperature is not critical and generally the reaction is carried out under cooling, at ordinary temperature, or under warming, for example, about -100°C to 100°C. The reaction time is about 30 minutes to 72 hours. The reaction solvent that can be conveniently employed includes aromatic hydrocarbons such as benzene, toluene, xylene, etc., halogenated hydrocarbons such as dichloromethane, chloroform, 1,2-dichloroethane, etc., and ethers such as diethyl ether, tetrahydrofuran, dioxane and so on. These solvents can be used singly or, if necessary, in a combination of two or more species. 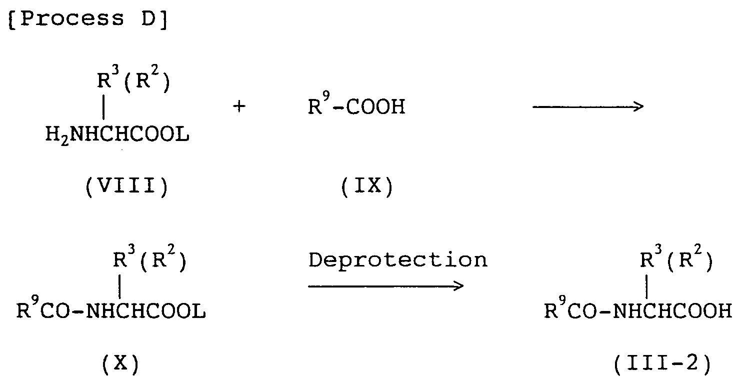 In the above reaction scheme, the respective symbols have the meanings defined hereinbefore.

In this process, compound (IX) or a reactive derivative of the carboxyl function thereof or a salt thereof is reacted with compound (VIII) or a reactive derivative of the amino function thereof or a salt thereof to produce compound (X) and, then, the carboxy-protecting group is then removed from compound (X) to give compound (III-2). This process is carried out in the same manner as Process C. 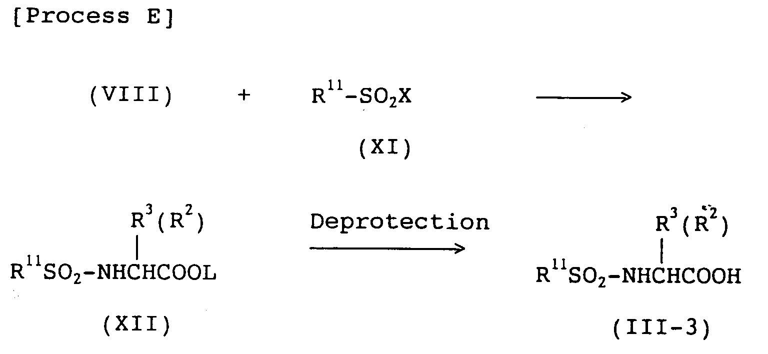 In the above reaction scheme, X is a halogen atom (e.g. chlorine, bromine) and the other symbols have the meanings defined hereinbefore.

In this process, compound (XI) or a salt thereof is reacted with compound (VIII) or a reactive derivative in the amino group thereof or a salt thereof to produce compound (XII) and, then, the carboxy-protecting group is eliminated to give compound (III-3). The reaction between compounds (VIII) and (XI) is conducted in a suitable solvent. The solvent that can be used includes but is not limited to aromatic hydrocarbons such as benzene, toluene, xylene, etc., ethers such as dioxane, tetrahydrofuran, dimethoxyethane, etc., ethyl acetate, acetonitrile, pyridine, N,N-dimethylformamide, dimethyl sulfoxide, chloroform, dichloromethane, 1,2-dichloroethane, 1,1,2,2-tetrachloroethane, acetone, 2-butanone, etc., and mixtures of such solvents. The reaction between compounds (VIII) and (XI) is carried out in the presence of a suitable base, e.g. alkali metal salts such as sodium hydroxide, potassium hydroxide, potassium carbonate, sodium carbonate, sodium hydrogen carbonate, etc., amines such as pyridine, triethylamine, N,N-dimethylaniline, etc., sodium hydride, and potassium hydride, among others. The amount of the base is preferably about 1-5 molar equivalents relative to compound (VIII). This reaction is generally conducted at a temperature of -20°C to 150°C, preferably about -10°C to 100°C. The reaction time is about 30 minutes to 72 hours. For the preferred salts of the compound (VIII), reference may be had to the acid addition salts mentioned for compound (I). For example, the salts include salts with inorganic acids and salts with organic acids. The preferred salts with inorganic acids are salts with hydrochloric acid, hydrobromic acid, nitric acid, sulfuric acid, phosphoric acid, etc. The preferred salts with organic acids are salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.

The resulting compound (XII) is subjected to deprotection reaction to provide compound (III-3). This deprotection reaction is carried out in the same manner as the deprotection reaction in Process C. 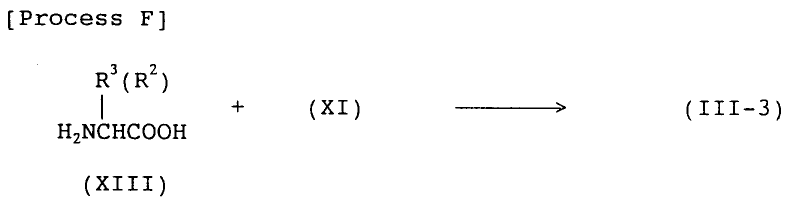 In the above reaction scheme, each symbol has the same meaning as defined hereinbefore.

According to this process, compound (XIII) or a salt thereof is reacted with compound (XI) or a salt thereof to give compound (III-3). This sulfonylation reaction is generally conducted in such a manner that the amino acid derivative (XIII) is first made into the sodium salt which is then reacted with compound (XI) and the reaction system is acidified, i.e. after the fashion of Schotten-Baumann reaction. For the preferred salt of the compound (XIII), reference may be had to the acid addition salts mentioned for compound (VIII). 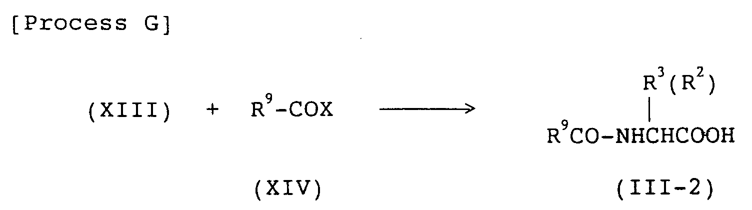 In the above reaction scheme, X is a halogen atom (e.g. chlorine, bromine) and the other symbols have the same meaning as defined hereinbefore.

According to this process, compound (XIII) or a salt thereof is reacted with compound (XIV) or a salt thereof to give compound (III-2). This acylation reaction is carried out in the same manner as Process F. 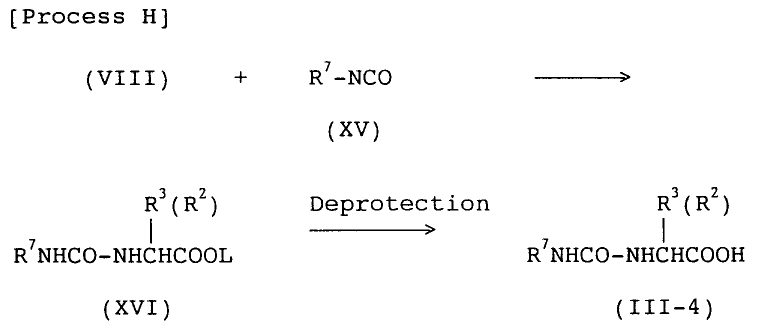 In the above reaction scheme, each symbol has the same meaning as defined hereinbefore.

According to this process, compound (VIII) or a salt thereof is reacted with compound (XV) to give (XVI) which is then subjected to the deprotection reaction of the carboxy-protecting group to give compound (III-4). The reaction between compound (VIII) or a salt thereof and compound (XV) is conducted in a suitable solvent. The solvent includes but is not limited to aromatic hydrocarbons such as benzene, toluene, xylene, etc., ethers such as dioxane, tetrahydrofuran, dimethoxyethane, etc., ethyl acetate, acetonitrile, pyridine, N,N-dimethylformamide, dimethyl sulfoxide, chloroform, dichloromethane, 1,2-dichloroethane, 1,1,2,2-tetrachloroethane, acetone, 2-butanone, etc., and their mixed solvents. The amount of compound (XV) is preferably about 1-5 molar equivalents relative to compound (VIII). This reaction is conducted generally at -20°C to 150°C and preferably at about - 10°C to 100°C. The reaction time is about 30 minutes to 72 hours. The resulting compound (XVI) is subjected to deprotection reaction to provide compound (III-4). This deprotection reaction is carried out in the same manner as the deprotection reaction in Process C. 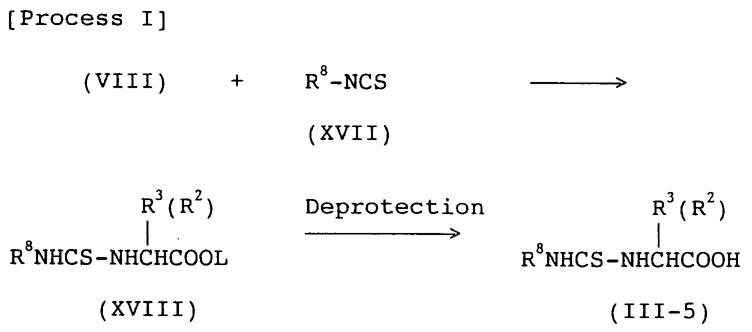 In the above reaction scheme, each symbol has the same meaning as defined hereinbefore.

According to this process, compound (VIII) or a salt thereof is reacted with compound (XVII) to give compound (XVIII) which is then subjected to a deprotection reaction of the carboxy-protecting group to give compound (III-5). This reaction is carried out in the same manner as Process H. 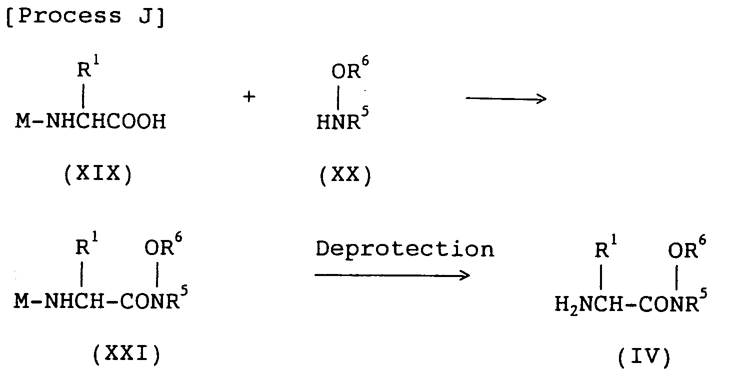 In the above reaction scheme, M represents an amino-protecting group; the other symbols have the same meanings as defined hereinbefore.

The amino-protecting group M includes a variety of protective groups in common usage in the field of peptide synthesis, e.g. acetyl, benzyloxycarbonyl, 4-methoxybenzyloxycarbonyl, t-butoxycarbonyl, and formyl, among others. Preferred are benzyloxycarbonyl and t-butoxycarbonyl.

This process comprises reacting compound (XIX) or a reactive derivative of the carboxyl function thereof or a salt thereof with compound (XX) or a reactive derivative of the amino function thereof or a salt thereof to give compound (XXI) and, then, subjecting (XXI) to the deprotection reaction of the amino protecting group to produce compound (IV). Deprotection of the amino function of compound (XXI) can be carried out by any routine procedure for amino deprotection. For example, benzyloxycarbonyl can be eliminated by catalytic reduction in the presence of the conventional metal catalyst (e.g. palladium-on-carbon, Raney nickel, etc.). The temperature for this reaction is not so critical and generally the reaction is carried out under cooling, at room temperature, or under warming, for example, about -100°C to 100°C . The reaction time is about 30 minutes to 72 hours. The reaction between compound (XIX) or a reactive derivative of the carboxyl function thereof or a salt thereof and compound (XX) or a reactive derivative of the amino function thereof or a salt thereof is carried out in the same manner as Process C.

The starting compound (I) for Process A can also be produced by the following process. 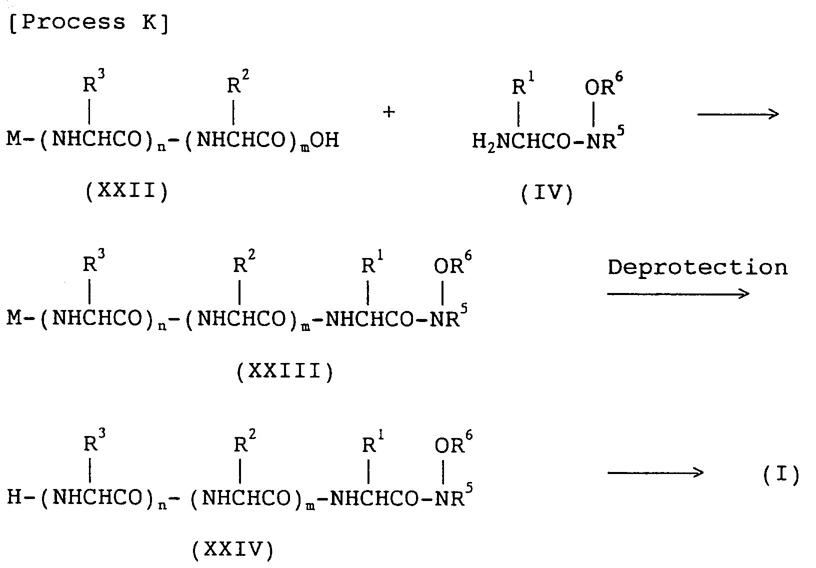 wherein M represents an amino-protecting group; the other symbols have the same meanings as defined hereinbefore.

The amino-protecting group M includes the species mentioned hereinbefore.

This process comprises reacting compound (XXII) or a reactive derivative of the carboxyl function thereof or a salt thereof with compound (IV) or a reactive derivative of the amino function thereof or a salt thereof to give compound (XXIII) and, then, subjecting compound (XXIII) to amino deprotection reaction to produce compound (XXIV). The reaction between compound (XXII) or a reactive derivative of the carboxyl function thereof or a salt thereof and compound (IV) or a reactive derivative of the amino function thereof or a salt thereof is carried out in the same manner as Process B. Deprotection of the amino protecting group of compound (XXIII) can be carried out by any routine procedure for removal of an amino-protecting group. For such procedures, reference can be had to the description of Process J. Then, compound (XXIV) is acylated in the same manner as the reaction between compounds (VIII) and (IX) in Process D or the reaction between compounds (XIII) and (XIV) in Process G, sulfonylated in the same manner as the reaction between compounds (VIII) and (II) in Process E, carbamoylated in the same manner as the reaction between compounds (VIII) and (XV) in Process H, or thiocarbamoylated in the same manner as the reaction between compounds (VIII) and (XVII) in Process I to give compound (I).

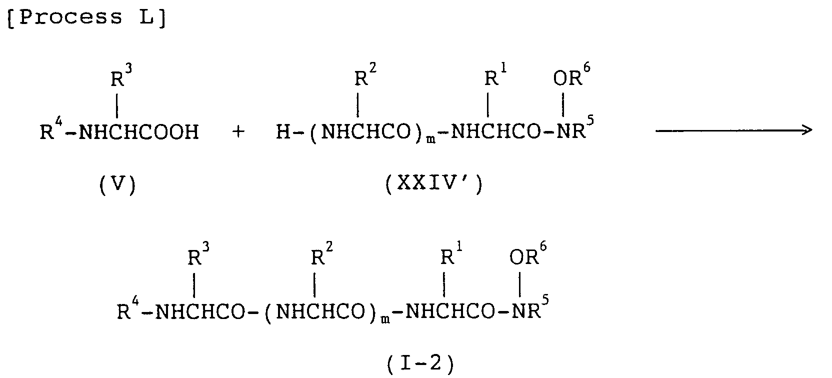

In the above reaction scheme, each symbol has the same meaning as defined hereinbefore.

In accordance with this process, compound (V) or a reactive derivative of the carboxyl function thereof or a salt thereof is reacted with compound (XXIV') or a reactive derivative of the amino function thereof or a salt thereof to produce compound (I-2). This reaction is carried out in the same manner as in Process C.

A process for producing a compound of the formula (II-1) 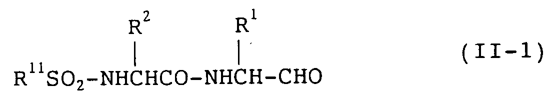 wherein each symbol has the same meaning as defined hereinbefore, preferably comprises
(i) reacting a compound of the formula (a) 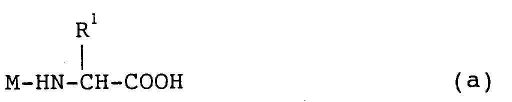 wherein R¹ and M are as defined above and or a reactive derivative of the carboxyl function thereof or a salt thereof with a compound of the formula (b) 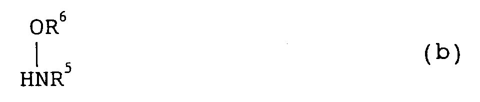 wherein R⁵ and R⁶ are the same meaning as defined hereinbefore or a reactive derivative of the amino function thereof or a salt thereof to give a compound of the formula (c) 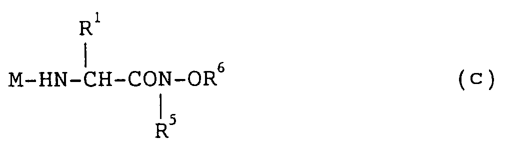 wherein R¹, R⁵, R⁶ and M are as defined above and, then, subjecting the compound to the deprotection reaction of the amino protecting group to produce a compound of the formula (d) 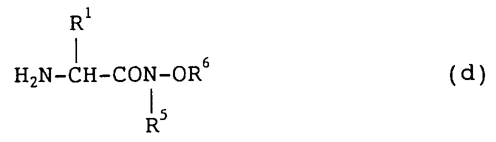 wherein R¹, R⁵, and R⁶ are as defined above,
(ii) reacting the compound of the formula (d) or a reactive derivative of the amino function thereof or a salt thereof with a compound of the formula (e) 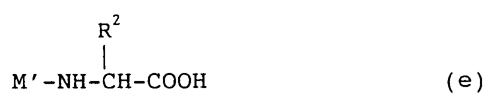 wherein R² is as defined above and M' represents an amino-protecting group or a reactive derivative of the carboxyl function thereof or a salt thereof to give a compound of the formula (f) 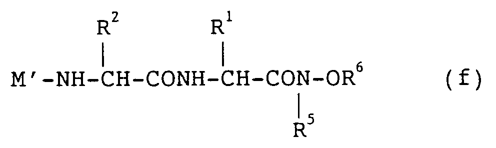 wherein R¹, R², R⁵, R⁶ and M' are as defined above and, then, subjecting the compound to the deprotection reaction of the amino-protecting group to produce a compound of the formula (g) 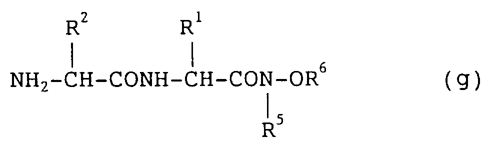 wherein R¹, R², R⁵ and R⁶ are as defined above,
(iii) reacting the compound of the formula (g) or a salt thereof with a compound of the formula (h)

   R¹¹SO₂X (h)

   wherein X is a halogen atom (e.g. chlorine, bromine) and R¹¹ is as defined above or a salt thereof to give a compound of the formula (i) 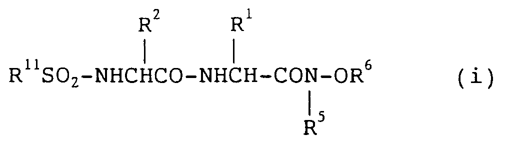 wherein each symbol is as defined above, and
(iv) subjecting the compound of the formula (i) or a salt thereof to a reduction reaction.

The amino-protecting group M' includes the same as defined in the amino-protecting group M.

The reaction between the compound (a) or a reactive derivative of the carboxyl function thereof or a salt thereof and the compound (b) or a reactive derivative of the amino function thereof or a salt thereof is carried out in the same manner as Process J. The deprotection reaction of the amino protecting group of the compound (c) can be carried out in the same manner as mentioned in Process J.

The reaction between the compound (d) or a reactive derivative of the amino function thereof or a salt thereof and the compound (e) or a reactive derivative of the carboxyl function thereof or a salt thereof is carried out in the same manner as Process K. The deprotection reaction of the amino protecting group of the compound (f) can be carried out in the same manner as in Process K.

The reaction between the compound (g) or a salt thereof and the compound (h) or a salt thereof is carried out in the same manner as Process E.

The reduction reaction of the compound (i) can be carried out in the same manner as in Process A.

The compound of the formula (II-1) is preferably a compound of

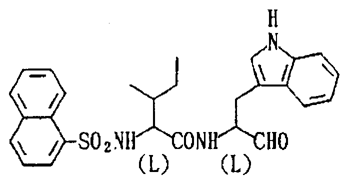

The method for producing a compound of the formula 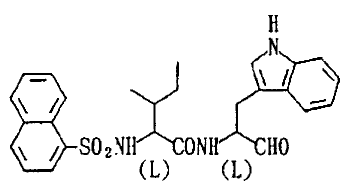 preferably comprises (i) reacting a compound of the formula (α) 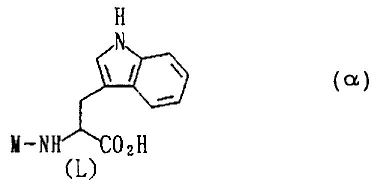 wherein M represents an amino-protecting group or a reactive derivative of the carboxyl function thereof or a salt thereof with a compound of the formula (β) 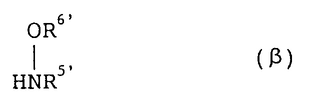 wherein R^{5'} and R^{6'} independently represent a C₁₋₆ alkyl group or a reactive derivative of the amino function thereof or a salt thereof to give a compound of the formula (γ) 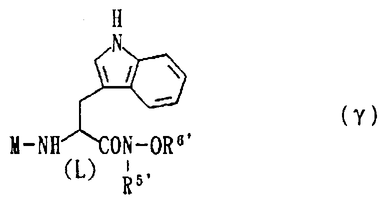 wherein R^{5'}, R^{6'} and M are as defined above and, then, subjecting the compound to the deprotection reaction of the amino protecting group to produce a compound of the formula (δ) 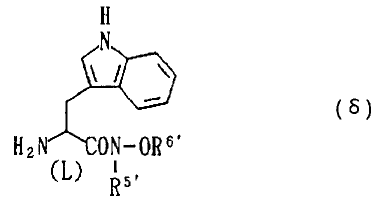 wherein R^{5'} and R^{6'} are as defined above,
(ii) reacting the compound of the formula (δ) or a reactive derivative of the amino function thereof or a salt thereof with a compound of the formula (ε) 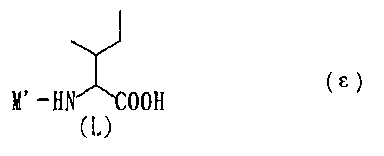 wherein M' represents an amino-protecting group or a reactive derivative of the carboxyl function thereof or a salt thereof to give a compound of the formula (ζ) 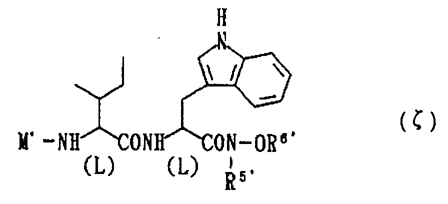 wherein R^{5'}, R^{6'} and M' are as defined above and, then, subjecting the compound to the deprotection reaction of the amino-protecting group to produce a compound of the formula (η) 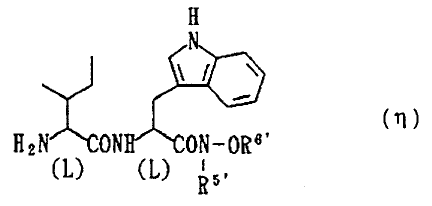 wherein R^{5'} and R^{6'} are as defined above,
(iii) reacting the compound of the formula (η) or a reactive derivative of the amino function thereof or a salt thereof with a compound of the formula (θ') 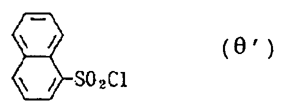 or a salt thereof to give a compound of the formula ( 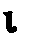 ) 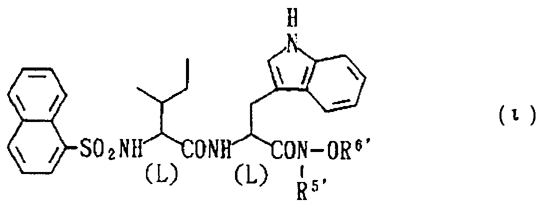 wherein R^{5'} and R^{6'} are as defined above, and
(iv) subjecting the compound of the formula ( 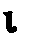 ) or a salt thereof to a reduction reaction.

In accordance with the production method of the present invention, an optically active compound (II) can be produced from compound (I) under mild conditions, in good yield and, in addition, without resort to DMSO oxidation reaction which is accompanied by evolution of a noxious odor. Therefore, the method of the invention is advantageous for commercial purposes as well.

The compound of the formula (II) as well as a salt thereof can be formulated with physiologically acceptable carriers to provide solid dosage forms, e.g. tablets, capsules, granules, powders, etc., or liquid dosage forms, e.g. syrups, injections, etc., each containing an effective amount for administration by the oral and other routes.

As disclosed in inter alia EP0611756, the compound of the formula (II) or a salt thereof has potent cathepsin L-inhibitory activity and bone resorption-inhibitory activity, with a low toxic potential. Therefore, the compound of the formula (II) and its salt can be used in the prophylaxis and therapy of osteoporosis in mammals (e.g. rat, mouse, rabbit, dog, cat, cattle, swine and man).

### Examples

The following reference and working examples are intended to describe the present invention in further detail and should by no means be construed as defining the scope of the invention. The room temperature is about 20°C to 30°C.

### Reference Example 1

N-benzyloxycarbonyl-L-tryptophan (40 g), N,O-dimethylhydroxylamine hydrochloride (12 g) and triethylamine (17.6 ml) were dissolved in dimethylformamide (DMF) (300 ml), followed by addition of 1-hydroxybenzotriazole (HOBt) (20 g) and 1-ethyl-3-(3-dimethylaminopropyl)carbodiimide hydrochloride (WSCD·HCl) (24.8 g) with ice-cooling, and the mixture was stirred at room temperature for 15 hours. This reaction mixture was concentrated under reduced pressure and the residue was diluted with ethyl acetate. The ethyl acetate layer was washed successively with 10% aqueous citric acid, water, aqueous saturated sodium hydrogen carbonate, and brine and dried (MgSO₄). The solvent was then distilled off and the residue was washed with ethyl acetate-hexane to provide N-benzyloxycarbonyl-L-tryptophan N,O-dimethylhydroxamide (42 g, 93%) as white solid. m.p. 131-132°C. [α]_{D}=-21.0° (c 0.56, MeOH) (20°C).

| Elemental analysis for C₂₁H₂₃N₃O₄ | | | |
|---|---|---|---|
| Calcd. | C, 66.13; | H, 6.08, | N, 11.02 |
| Found | C, 66.38; | H, 6.28, | N, 11.25 |

### Reference Example 2

The procedure of Reference Example 1 was virtually repeated to provide N-benzyloxycarbonyl-L-alanine N,O-dimethylhydroxamide as colorless prisms. m.p. 88-89°C. [α]_{D}=-16.6° (c 0.89, MeOH) (20°C).

| Elemental analysis for C₁₃H₁₈N₂O₄ | | | |
|---|---|---|---|
| Calcd. | C, 58.63; | H, 6.81, | N, 10.52 |
| Found | C, 58.59; | H, 6.71, | N, 10.51 |

### Reference Example 3

N-benzyloxycarbonyl-L-tryptophan N,O-dimethylhydroxamide (35 g) and palladium-on-carbon (5%, 18 g) were added to a mixed solvent consisting of methanol (200 ml) and tetrahydrofuran (THF) (200 ml) and a catalytic hydrogenation was carried out at atmospheric pressure, and room temperature. The palladium-on-carbon was filtered off and the filtrate was concentrated under reduced pressure. The residue was dissolved in dimethylformamide (DMF) (250 ml), followed by addition of N-benzyloxycarbonyl-L-isoleucine (25.5 g). To this solution was added 1-hydroxybenzotriazole (HOBt) (15.4 g) as well as 1-ethyl-3-(3-dimethylaminopropyl)carbodiimide hydrochloride (WSCD·HCl) (19.3 g) with ice-cooling, and the mixture was stirred at room temperature for 15 hours. This reaction mixture was concentrated under reduced pressure and the residue was diluted with ethyl acetate. The ethyl acetate layer was washed successively with 10% aqueous citric acid, water, aqueous saturated sodium hydrogen carbonate, and brine and dried (MgSO₄). The solvent was then distilled off and the residue was subjected to silica gel column chromatography. The fraction eluted out using ethyl acetate-hexane (3:1) yielded N-benzyloxycarbonyl-L-isoleucyl-L-tryptophan N,O-dimethylhydroxamide (42 g, 92%) as colorless powder. [α]_{D}=-36.6° (c 0.55, MeOH) (20°C).

| Elemental analysis for C₂₇H₃₄N₄O₅·1/2H₂O | | | |
|---|---|---|---|
| Calcd. | C, 64.40; | H, 7.01, | N, 11.13 |
| Found | C, 64.49; | H, 6.77, | N, 11.30 |

### Reference Example 4

N-benzyloxycarbonyl-L-isoleucyl-L-tryptophan N,O-dimethylhydroxamide (42 g) and palladium-on-carbon (5%, 18 g) were added to a mixed solvent consisting of methanol (150 ml) and tetrahydrofuran (THF) (150 ml) and a catalytic reduction was carried out at atmospheric pressure and room temperature. The palladium-on-carbon was then filtered off and the filtrate was concentrated under reduced pressure. The residue was dissolved in dimethylformamide (DMF) (300 ml) followed by addition of 1-naphthalenesulfonyl chloride (20.2 g) and N,N-dimethylaminopyridine (10.9 g) with ice-cooling, and the mixture was stirred at the same temperature for 3 hours. This reaction mixture was concentrated under reduced pressure and the residue was diluted with ethyl acetate. The ethyl acetate layer was washed successively with 10% aqueous citric acid, water, aqueous saturated sodium hydrogen carbonate, and brine and dried (MgSO₄). The solvent was then distilled off and the residue was subjected to silica gel column chromatography. The fraction eluted out using ethyl acetate-hexane (3:1) yielded N-(1-naphthalenesulfonyl)-L-isoleucyl-L-tryptophan N,O-dimethylhydroxamide (42 g, 83%) as light-yellow powder. To this powder was added toluene and the mixture was concentrated under reduced pressure to remove the ethyl acetate thoroughly. [α]_{D}=+30.2° (c 0.75, MeOH) (20°C).

| Elemental analysis for C₂₉H₃₄N₄O₅S·1/2 toluene | | | |
|---|---|---|---|
| Calcd. | C, 65.41; | H, 6.42, | N, 9.39 |
| Found | C, 65.44; | H, 6.43, | N, 9.26 |

### Reference Example 5

The procedure of Reference Example 3 was virtually repeated to provide N-benzyloxycarbonyl-L-valyl-L-phenylalanine N,O-dimethylhydroxamide as colorless crystals. m.p. 101-102°C. [α]_{D}=-36.9° (c 0.76, MeOH) (20°C).

| Elemental analysis for C₂₄H₃₁N₃O₅ | | | |
|---|---|---|---|
| Calcd. | C, 65.29; | H, 7.08, | N, 9.52 |
| Found | C, 65.08; | H, 7.05, | N, 9.41 |

From the above product, colorless needles of N-valproyl-L-valyl-L-phenylalanine N,O-dimethylhydroxamide were obtained. m.p. 157-158°C. [α]_{D}=-54.6° (c 0.66, MeOH) (20°C).

| Elemental analysis for C₂₄H₃₉N₃O₄ | | | |
|---|---|---|---|
| Calcd. | C, 66.48; | H, 9.07, | N, 9.69 |
| Found | C, 66.43; | H, 9.04, | N, 9.86 |

### Reference Example 6

The procedure of Reference Example 3 was virtually repeated to provide N-(α-t-butoxycarbonyl)-N-(ε-benzyloxycarbonyl)-L-lysyl-L-alanine N,O-dimethylhydroxamide as a colorless oil.

¹H-NMR (δ ppm in CDCl₃): 1.33 (3H, d, J=7.0Hz), 1.44 (9H, s), 1.5-1.9 (6H, m), 3.13 (3H, s), 3.1-3.3 (2H, m), 3.75 (3H, s), 4.0-4.2 (1H, m), 4.8-5.0 (1H, m), 5.09 (2H, s), 5.1-5.3 (1H, m), 6.63 (1H, d, J=7.4Hz), 7.35 (5H, s).

### Reference Example 7

The procedure of Reference Example 4 was virtually repeated to provide N-α-(p-toluenesulfonyl)-N-(ε-benzyloxycarbonyl)-L-lysyl-L-alanine N,O-dimethylhydroxamide as colorless needles. m.p. 102-103°C. [α]_{D}=-25.5° (c 0.55, MeOH) (20°C).

| Elemental analysis for C₂₆H₃₆N₄O₇S | | | |
|---|---|---|---|
| Calcd. | C, 56.92; | H, 6.61, | N, 10.21 |
| Found | C, 57.05; | H, 6.54, | N, 10.19 |

### Example 1

N-(1-naphthalenesulfonyl)-L-isoleucyl-L-tryptophan N,O-dimethylhydroxamide (21 g) was dissolved in dry tetrahydrofuran (THF) (200 ml) and the solution was cooled to -60°C in N₂ streams. To this solution was added 1.5M diisobutylaluminum hydride-toluene (107 ml) dropwise over a period of 25 minutes. This mixture was stirred at -50°C for 4 hours, at the end of which time it was poured in an aqueous solution of citric acid and extracted with ethyl acetate. The extract was washed successively with aqueous citric acid, water, aqueous saturated sodium hydrogen carbonate, and brine and, then, dried (MgSO₄). The solvent was distilled off and the residue was crystallized from ethyl acetate-hexane to provide N-(1-naphthalenesulfonyl)-L-isoleucyl-L-tryptophanal (9.0 g, 58%) as colorless crystals. m.p. 156-157°C. [α]_{D}=-54.4° (c 0.50, CHCl₃) (20°C).

| Elemental analysis for C₂₇H₂₉N₃O₄S | | | |
|---|---|---|---|
| Calcd. | C, 65.97; | H, 5.95, | N, 8.55 |
| Found | C, 65.95; | H, 6.16, | N, 8.36 |

### Example 2

The procedure of Example 1 was virtually repeated to provide N-(valproyl)-L-valyl-L-phenylalaninal as colorless crystals. m.p. 161-162°C. [α]_{D}=-59.3° (c 0.84, DMSO) (20°C).

| Elemental analysis for C₂₂H₃₄N₂O₃ | | | |
|---|---|---|---|
| Calcd. | C, 70.55; | H, 9.15, | N, 7.48 |
| Found | C, 70.16; | H, 9.13, | N, 7.76 |

### Example 3

The procedure of Example 1 was virtually repeated to provide N-α-(p-toluenesulfonyl)-N-(ε-benzyloxycarbonyl)-L-lysyl-L-alaninal as colorless crystals. m.p. 161-162°C. [α]_{D}=-9.2° (c 0.53, MeOH).

| Elemental analysis for C₂₄H₃₁N₃O₆S | | | |
|---|---|---|---|
| Calcd. | C, 58.88; | H, 6.38, | N, 8.58 |
| Found | C, 58.46; | H, 6.36, | N, 8.61 |

The invention has been described in detail with reference to preferred embodiments thereof. However, it will be appreciated that those skilled in the art, upon consideration of this disclosure, may make modifications and improvements within the spirit and scope of the invention.

## Claims

1. A method for producing a compound of the formula (II) 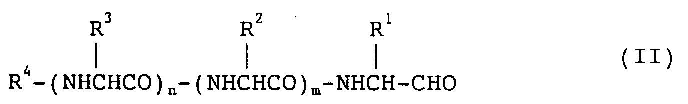 wherein R¹, R² and R³ independently represent hydrogen or a hydrocarbon group that may be substituted; R⁴ represents acyl; and m and n are independently 0 or 1 or a salt thereof, which comprises subjecting a compound of the formula (I) 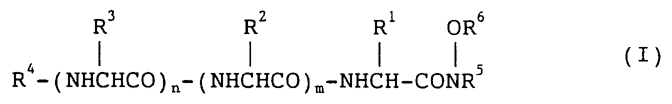 wherein R¹, R², R³, R⁴, m and n are as defined above; R⁵ and R⁶ independently represent a hydrocarbon group that may be substituted, or a salt thereof to a reduction reaction.

2. A method claimed in Claim 1, in which the reduction reaction is carried out using an aluminum hydride type reducing agent.

3. A method claimed in Claim 1, in which R₁, R₂ and R³ independently represent hydrogen or a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl wherein the hydrocarbon group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which may be substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen or a 5-membered ring containing 1 atom of sulfur in which the heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy; R⁴ represents a group of the formula -CONHR⁷, -CSNHR⁸, -COR⁹, -SOR¹⁰ or -SO₂R¹¹ wherein R⁷, R⁸, R⁹, R¹⁰ and R¹¹ independently represent a hydrogen atom or (A) a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl or (B) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur and oxgen which may be condensed with a 6-membered ring containing 2 or fewer atoms of nitrogen, a benzene ring or a 5-membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may be condensed with a benzene ring, a 6-membered ring containing 2 or fewer atoms of nitrogen, or a 5-membered ring containing 1 atom of sulfur which hydrocarbon or heterocyclic group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which may be substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen, or a 5-membered ring containing 1 atom of sulfur which heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy; R⁵ and R⁶ independently represent hydrogen or a hydrocarbon group selected from the group consisting of C₁₋₁₀ alkyl, C₂₋₁₀ alkenyl, C₂₋₁₀ alkynyl, C₃₋₁₂ cycloalkyl, C₅₋₁₂ cycloalkenyl, C₅₋₁₂ cycloalkadienyl, C₃₋₇ cycloalkyl-C₁₋₈ alkyl, C₅₋₇ cycloalkenyl-C₁₋₈ alkyl and C₆₋₁₄ aryl wherein the hydrocarbon group may have 1 to 3 substituents selected from the group consisting of (i) a C₆₋₁₄ aryl group which may be substituted with hydroxy, C₁₋₃ alkoxy, halogen or C₁₋₃ alkyl, (ii) a C₃₋₇ cycloalkyl or C₃₋₆ cycloalkenyl group which may be substituted with hydroxy, C₁₋₃ alkoxy, halogen or C₁₋₃ alkyl, (iii) a heterocyclic group selected from the group consisting of a 5- to 7-membered aromatic heterocyclic group containing 1 atom of sulfur, nitrogen or oxgen, 5- or 6-membered aromatic heterocyclic group containing 2 to 4 atoms of nitrogen or 5- or 6-membered aromatic heterocyclic group containing 1 or 2 atoms of nitrogen and 1 atom of sulfur or oxgen which may condense with a 6-membered ring containing 2 or fewer atoms of nitrogen, a benzene ring or a 5- membered ring containing 1 atom of sulfur and a 5- to 7-membered non-aromatic heterocyclic group containing 1 atom of sulfur, nitrogen or oxgen or 4- to 7-membered non-aromatic heterocyclic group containing 1 atom of nitrogen and 3 or fewer atoms selected from nitrogen, oxgen and sulfur which may condense with a benzene ring, a 6-membered ring containing 2 or fewer atoms of nitrogen, or a 5-membered ring containing 1 atom of sulfur which heterocyclic group may be substituted with C₁₋₃ alkyl, (iv) carboxy, (C₁₋₆ alkoxy)carbonyl, (C₆₋₁₀ aryloxy)carbonyl or (C₇₋₁₃ aralkyloxy)carbonyl, (v) a carbamoyl group which may be substituted with C₁₋₆ alkyl, C₃₋₆ cycloalkyl, C₆₋₁₀ aryl or C₇₋₁₃ aralkyl, (vi) an amino group which may be substituted with C₁₋₆ alkyl, C₃₋₆ cycloalkyl, C₆₋₁₀ aryl or C₇₋₁₃ aralkyl, (vii) a hydroxyl group which may be substituted with C₁₋₆ alkyl, C₃₋₆ cycloalkyl, C₆₋₁₀ aryl or C₇₋₁₃ aralkyl, (viii) a thiol group which may be substituted with C₁₋₆ alkyl, C₃₋₆ cycloalkyl, C₆₋₁₀ aryl or C₇₋₁₃ aralkyl, (ix) halogen and (x) a phosphono group which may be substituted with C₁₋₆ alkyl or C₁₋₆ alkoxy.

4. A method claimed in Claim 1, in which R¹, R² and R³ are independently an optionally substituted alkyl group.

5. A method claimed in Claim 1, in which R¹ is a straight-chain or branched C₁₋₆ alkyl group which is substituted with an aryl group or a heterocyclic group.

6. A method claimed in Claim 1, in which R² and R³ are independently a straight-chain or branched C₁₋₆ alkyl group.

7. A method claimed in Claim 1, in which the acyl group is that derived from a carboxylic acid, sulfonic acid, sulfinic acid, carbamic acid or thiocarbamic acid.

8. A method claimed in Claim 1, in which the acyl group is represented by the formula -SO₂R¹¹ or -COR⁹, wherein R¹¹ and R⁹ are independently a hydrogen atom or an optionally substituted hydrocarbon or heterocyclic group.

9. A method claimed in Claim 1, in which R⁴ represents a group of the formula -SO₂R^{11'} wherein R^{11'} is a C₆₋₁₀ aryl group.

10. A method claimed in Claim 1, in which R⁵ and R⁶ independently represent a saturated or unsaturated aliphatic acyclic hydrocarbon group.

11. A method claimed in Claim 10, in which the saturated aliphatic hydrocarbon group is a C₁₋₁₀ alkyl group.

12. A method claimed in Claim 11 wherein the alkyl is methyl.

13. A method claimed in claim 1, in which m is 1 and n is 0.

14. A method claimed in claim 1, in which the compound of the formula (I) is one of the formula ( 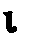 ) 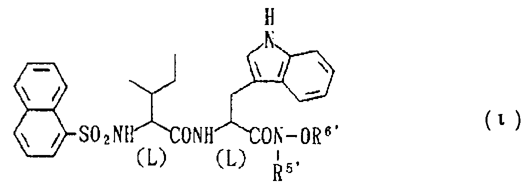 wherein R^{5'} and R^{6'} independently a C₁₋₆ alkyl group or a reactive derivative of the amino function thereof, or a salt thereof.

15. A compound of the formula (I) 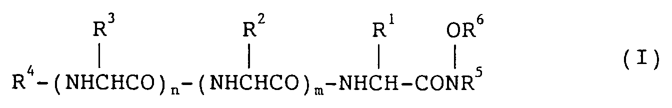 wherein R¹, R² and R³ independently represent hydrogen or a hydrocarbon group that may be substituted; R⁴ represents acyl; R⁵ and R⁶ independently represent a hydrocarbon group that may be substituted; and m and n are independently 0 or 1, or a salt thereof.

16. A compound claimed in claim 13, which is one of the formula ( 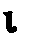 ) 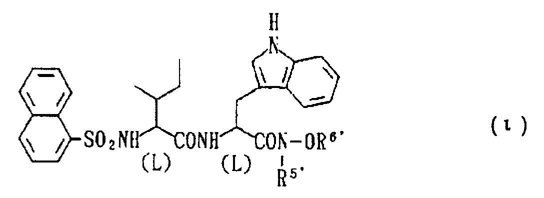 wherein R^{5'} and R^{6'} independently a C₁₋₆ alkyl group or a reactive derivative of the amino function thereof, or a salt thereof.

17. A method for producing a compound of the formula 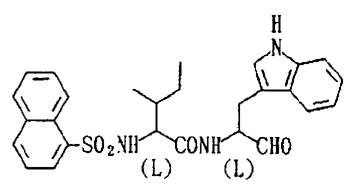 which comprises (i) reacting a compound of the formula (α) 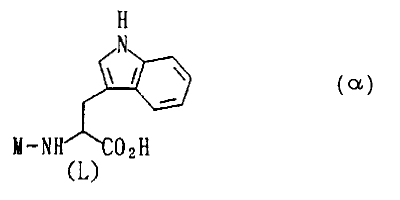 wherein M represents an amino-protecting group, or a reactive derivative of the carboxyl function thereof or a salt thereof with a compound of the formula (β) 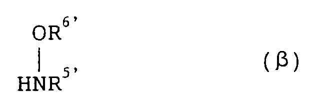 wherein R^{5'} and R^{6'} independently represent a C₁₋₆ alkyl group, or a reactive derivative of the amino function thereof or a salt thereof to give a compound of the formula (γ) 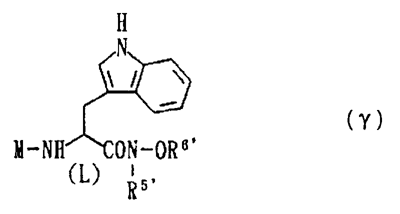 wherein R^{5'}, R^{6'} and M are as defined above and, then, subjecting a compound of the formula (γ) to the deprotection reaction of the amino protecting group to produce a compound of the formula (δ) 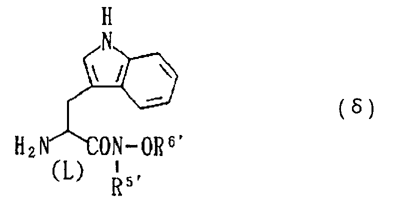 wherein R^{5'} and R^{6'} are as defined above,
(ii) reacting the compound of the formula (δ) or a reactive derivative of the amino function thereof or a salt thereof with a compound of the formula (ε) 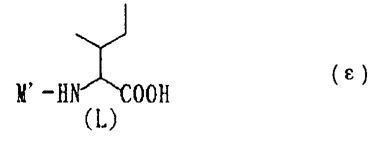 wherein M' represents an amino-protecting group or a reactive derivative of the carboxyl function thereof or a salt thereof to give a compound of the formula (ζ) 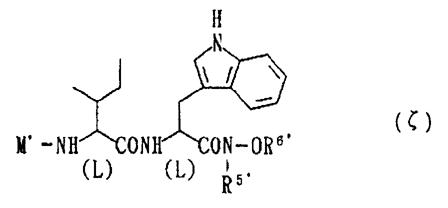 wherein R^{5'}, R^{6'} and M' are as defined above and, then, subjecting a compound of the formula (ζ) to the deprotection reaction of the amino-protecting group to produce a compound of the formula (η) 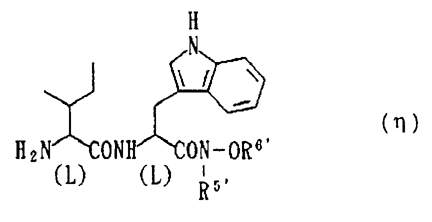 wherein R^{5'} and R^{6'} are as defined above,
(iii) reacting the compound of the formula (η) or a reactive derivative of the amino function thereof or a salt thereof with a compound of the formula (θ) 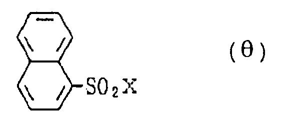 wherein X is a halogen atom, or a salt thereof to give a compound of the formula ( 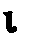 ) 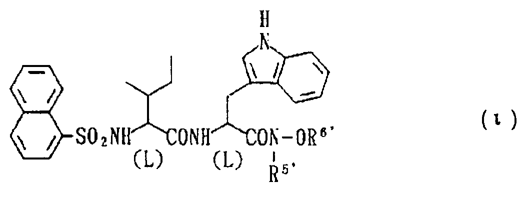 wherein R^{5'} and R^{6'} are as defined above, and
(iv) subjecting the compound of the formula ( 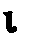 ) or a salt thereof to reduction reaction.
